(19) Europäisches Patentamt
European Patent Office
Office européen des brevets

(11) EP 0 859 590 B1

# (12) FASCICULE DE BREVET EUROPEEN

(45) Date de publication et mention de la délivrance du brevet:
**19.12.2001 Bulletin 2001/51**

(21) Numéro de dépôt: **96931851.8**

(22) Date de dépôt: **16.09.1996**

(51) Int Cl.[7]: **A61K 7/48**, A61K 7/06

(86) Numéro de dépôt international:
**PCT/FR96/01433**

(87) Numéro de publication internationale:
**WO 97/12593 (10.04.1997 Gazette 1997/16)**

(54) **COMPOSITION COSMETIQUE COMPRENANT AU MOINS UN POLYMERE SILICONE GREFFE ET AU MOINS UN COPOLYMERE BLOC LINEAIRE POLYSILOXANE-POLYOXYALKYLENE**

EINE MINDESTENS EIN GEPFROPFTES SILIKONPOLYMER UND MINDESTENS EIN LINEARES POLYSILOXANPOLYALKYLEN BLOCKPOLYMER ENTHALTENDE KOSMETISCHE ZUBEREITUNG

COSMETIC COMPOSITION INCLUDING AT LEAST ONE SILICONE-GRAFTED POLYMER AND AT LEAST ONE POLYSILOXANE-POLYOXYALKYLENE LINEAR BLOCK COPOLYMER

(84) Etats contractants désignés:
**AT BE CH DE DK ES FI FR GB GR IE IT LI LU MC NL PT SE**

(30) Priorité: **29.09.1995 FR 9511479**

(43) Date de publication de la demande:
**26.08.1998 Bulletin 1998/35**

(73) Titulaire: **L'OREAL**
**75008 Paris (FR)**

(72) Inventeurs:
- **DUBIEF, Claude**
  **F-78150 Le Chesnay (FR)**
- **DUPUIS, Christine**
  **F-75018 Paris (FR)**
- **CAUWET-MARTIN, Danièle**
  **F-75011 Paris (FR)**

(74) Mandataire: **Le Blainvaux Bellegarde, Françoise**
**L'OREAL-DPI 6 rue Bertrand Sincholle**
**92585 Clichy Cedex (FR)**

(56) Documents cités:
**EP-A- 0 125 779**
**EP-A- 0 412 704**
**EP-A- 0 412 707**
**EP-A- 0 492 657**
**EP-A- 0 582 152**
**EP-A- 0 635 258**
**EP-A- 0 643 961**
**WO-A-93/23009**
**WO-A-93/23446**
**WO-A-95/03776**
**FR-A- 2 709 955**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 859 590 B1

## Description

La présente invention a trait à une composition cosmétique ou dermatologique pour le traitement des matières kératiniques, en particulier des cheveux humains comprenant au moins un polymère siliconé greffé et au moins un copolymère ayant comme unités répétitives un bloc linéaire polysiloxane-polyoxyalkylène.

On connaît dans l'état de la technique des polymères siliconé greffé tels que ceux décrits dans les demandes de brevet EP-A-0 412 704, EP-A-0 412 707, EP-A-0 640 105 et WO 95/00578, EP-A-0582 152 et WO 93/23009. Ces polymères sont utilisés en capillaire pour leurs propriétés coiffantes. Cependant, lorsque l'on utilise ces polymères, le pouvoir fixant, la tenue de la coiffure et le toucher des cheveux ne sont pas encore satisfaisants.

Par pouvoir fixant de la composition on désignera l'aptitude de cette dernière à donner aux cheveux une cohésion telle que la mise en forme initiale de la coiffure est conservée.

On connaît des silicones possédant des chaînes polyoxyalkylène greffées encore appelées selon la nomenclature CTFA Dimethicone copolyol. La demanderesse a constaté que ces silicones diminuaient le pouvoir fixant des compositions pour le maintien de la coiffure.

La demanderesse a découvert de façon surprenante qu'en associant au moins un polymère siliconé greffé avec au moins un copolymère ayant un bloc linéaire polysiloxane-polyoxyalkylène comme unités répétitives, le pouvoir fixant des compositions et le toucher des cheveux étaient sensiblement supérieures à ceux obtenus avec le polymère siliconé greffé utilisé seul.

La composition selon l'invention est donc essentiellement caractérisée par le fait qu'elle contient dans un milieu cosmétiquement ou dermatologiquement acceptable au moins un polymère siliconé greffé comprenant une portion polysiloxane et une portion constituée d'une chaîne organique non-siliconée, l'une des deux portions constituant la chaîne principale du polymère l'autre étant greffée sur la dite chaîne principale et au moins un copolymère ayant comme unités répétitives un bloc linéaire polysiloxane-polyoxyalkylène.

Les polymères siliconés greffés selon l'invention sont choisis préférentiellement parmi les polymères à squelette organique non siliconé greffé par des monomères contenant un polysiloxane, les polymères à squelette polysiloxanique greffé par des monomères organiques non siliconés et leurs mélanges.

Dans ce qui suit, on entend désigner par silicone ou polysiloxane, en conformité avec l'acception générale, tous les polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en $C_1$-$C_{10}$ et en particulier méthyle, les radicaux fluoroalkyls, les radicaux aryls et en particulier phényle, et les radicaux alcényls et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quatemaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Dans ce qui suit, on entend désigner par «macromère polysiloxane», en conformité avec l'acception générale, tout monomère contenant dans sa structure une chaîne polymère du type polysiloxane.

Les polymères à squelette organique non-siliconé greffé par des monomères contenant un polysiloxane conformes à la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane conformes à la présente invention peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs composés organiques non siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté

sur une des extrémités de la silicone avec une double liaison d'un monomère à insaturation éthylénique de la chaîne principale.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :

a) de 0 à 98% en poids d'au moins un monomère (A) lipophile de faible polarité lipophile à insaturation éthylénique, polymérisable par voie radicalaire ;
b) de 0 à 98% en poids d'au moins un monomère (B) hydrophile polaire à insaturation éthylénique, copolymérisable avec le ou les monomères du type (A) ;
c) de 0,01 à 50 % en poids d'au moins un macromère polysiloxane (C) de formule générale :

$$X(Y)_nSi(R)_{3-m}\,Z_m \qquad (I)$$

où :

X désigne un groupe vinylique copolymérisable avec les monomères (A) et (B) ;
Y désigne un groupe de liaison divalent ;
R désigne un hydrogène, un alkyle ou un alcoxy en $C_1$-$C_6$, un aryle $C_6$-$C_{12}$ ;
Z désigne un motif polysiloxane monovalent ayant un poids moléculaire moyen en nombre d'au moins 500 ;
n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,693,935, US 4,728,571 et US 4,972,037 et les demandes de brevet EP-A-0 412 704, EP-A-0 412 707, EP-A-0 640 105. Ils ont un poids moléculaire moyen en nombre de préférence allant de 10.000 à 2.000.000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en $C_1$-$C_{18}$ ; le styrène ; les macromères polystyrène ; l'acétate de vinyle ; le propionate de vinyle ; l'alpha-méthylstyrène ; le tertio-butylstyrène; le butadiène ; le cyclohexadiène ; le cyclohexadiène ; l'éthylène ; le propylène ; le vinyltoluène ; les esters d'acide acrylique ou méthacrylique et de 1,1-dihydroperfluoroalcanols ou de leurs homologues ; les esters d'acide acrylique ou méthacrylique et de oméga-hydrydofluroalcanols ; les esters d'acide acrylique ou méthacrylique et de fluoroalkylsulfoamido-alcools; les esters d'acide acrylique ou méthacrylique et d'alcools fluoroalkyliques ; les esters d'acide acrylique ou méthacrylique et de fluroéthers d'alcools ; ou leurs mélanges. Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perfluro-octane sulfonamido)-éthylacrylate ; le 2-(N-butylperfluorooctane sulfonamido)-éthylacrylate ou leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quatemisé, le (méth)acrylamide, le N-t-butylacrylamide, l'acide maléique, l'anhydride maléique et leurs demi-esters, les (méth) acrylates hydroxyalkylés, le chlorure de diallyldiméthylammonium, la vinyl-pyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinyl caprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

Les macromères polysiloxane (C) de formule (I) préférentiels sont choisis parmi ceux répondant à la formule générale suivante (II) :

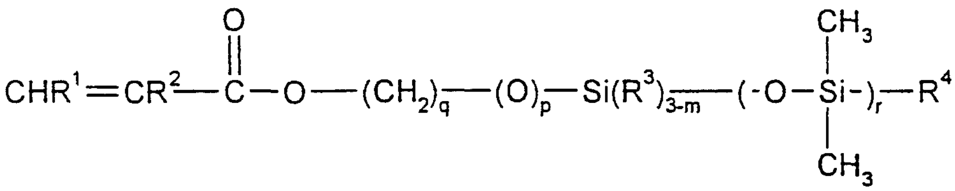 (II)

dans laquelle :

- $R^1$ est hydrogène ou -COOH (de préférence hydrogène) ;
- $R^2$ est hydrogène, méthyle ou $-CH_2COOH$ (de préférence méthyle) ;
- $R^3$ est alkyle, alcoxy ou alkylamino en $C_1$-$C_6$, aryle en $C_6$-$C_{12}$ ou hydroxyle (de préférence méthyle) ;
- $R^4$ est alkyle, alcoxy ou alkylamino en $C_1$-$C_6$, aryle en $C_6$-$C_{12}$ ou hydroxyle (de préférence méthyle) ;
- q est un entier de 2 à 6 (de préférence 3) ;
- p est 0 ou 1 ;
- r est un nombre entier de 5 à 700 ;
- m est un entier allant de 1 à 3 (de préférence 1) ;

On utilise plus particulièrement les macromères polysiloxanes de formule :

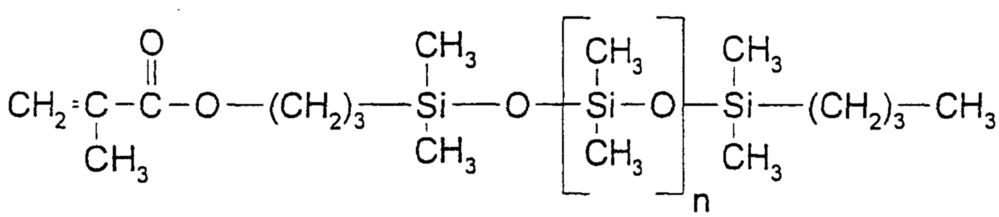

avec n étant un nombre allant de 5 à 700.

Un mode particulier de réalisation de l'invention consiste à utiliser un copolymère susceptible d'être obtenu par polymérisation radicalaire à partir du mélange de monomères constitué par :

a) 60% en poids d'acrylate de tertiobutyle ;
b) 20% en poids d'acide acrylique ;
c) 20% en poids de macromère siliconé de formule :

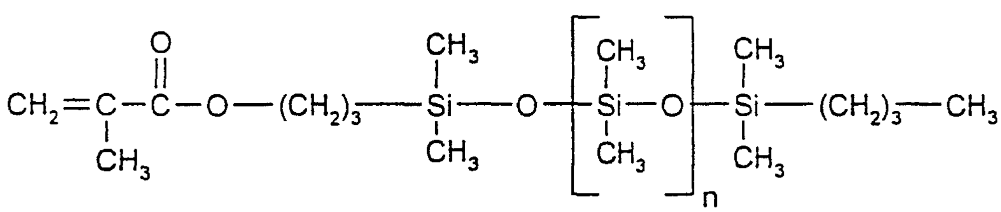

avec n étant un nombre allant de 5 à 700 ; les pourcentages en poids étant calculés par rapport au poids total des monomères.

Un autre mode particulier de réalisation de l'invention consiste à utiliser un copolymère susceptible d'être obtenu par polymérisation radicalaire à partir du mélange de monomères constitué par :

a) 80% en poids d'acrylate de tertiobutyle ;
b) 20% en poids de macromère siliconé de formule :

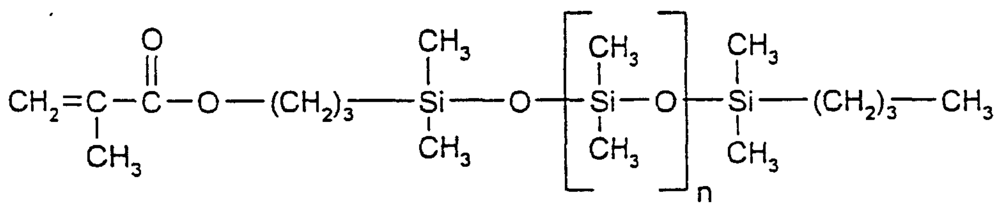

avec n étant un nombre allant de 5 à 700 ; les pourcentages en poids étant calculés par rapport au poids total des monomères.

Une autre famille particulière de polymères siliconés greffés à squelette organique non siliconé convenant pour la réalisation de la présente invention est constituée par les copolymères greffés siliconés susceptible d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que propylène, styrène, alkyl styrène, butylène, butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth) acrylique ; ceux comportant une fonction ester tels que les esters de l'acide (méth) acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires. et plus particulièrement parmi ceux répondant à la formule générale :

$$T\text{-}(CH_2)_s\text{-}Si\text{-}[\text{-}(OSiR^5R^6)_t\text{-}R^7]_y \qquad (III)$$

dans laquelle T est choisi dans le groupe constitué par NH2, NHR', une fonction époxy, OH, SH ; $R^5$, $R^6$, $R^7$ et R', indépendamment, désignent un alkyle en $C_1$-$C_6$, phényle, benzyle, ou alkylphényle en $C_6$-$C_{12}$, hydrogène ; s est un nombre de 2 à 00 ; t est un nombre de 0 à 1000 et y est un nombre de 1 à 3. Ils ont un poids moléculaire moyen en nombre de préférence allant de 5.000 à 300.000, plus préférentiellement de 8.000 à 200.000 et plus particulièrement de 9000 à 40.000.

Selon la présente invention, le ou les polymères siliconés greffés, à squelette polysiloxanique greffé par des monomères organiques non siliconés comprennent une chaîne principale de silicone (ou polysiloxane $(\equiv Si\text{-}O\text{-})_n$) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements $\equiv$Si-H et des groupements vinyliques $CH_2$=CH-, ou encore la réaction entre des groupements thio-fonctionnels -SH avec ces mêmes groupements vinyliques.

Des exemples de polymères à squelette polysiloxanique greffé par des monomères organiques non siliconés convenant à la mise en oeuvre de la présente invention, ainsi que leur mode particulier de préparation, sont notamment décrits dans les demandes de brevets EP-A-0 582 162, WO 93/23009 et WO 95/03776 dont les enseignements sont totalement inclus dans la présente description à titre de références non limitatives.

Selon un mode particulièrement préféré de réalisation de la présente invention, le polymère siliconé, à squelette polysiloxanique greffé par des monomères organiques non siliconés, mis en oeuvre comprend le résultat de la copolymérisation radicalaire entre d'une part au moins un monomère organique anionique non-siliconé présentant une

insaturation éthylénique et/ou un monomère organique hydrophobe non-siliconé présentant une insaturation éthylénique et d'autre part une silicone présentant dans sa chaîne au moins un, et de préférence plusieurs, groupements fonctionnels capables de venir réagir sur lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou en mélange, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélange, parmi les esters d'acide acrylique alcanols et/ou les esters d'acide méthacrylique d'alcanols. Les alcanols sont de préférence en $C_1$-$C_{18}$ et plus particulièrement en $C_1$-$C_{12}$. Les monomères préférentiels sont choisis dans le groupe constitué par le (méth)acrylate d'isooctyle, le (méth)acrylate d'isononyle, le 2-éthylhexyl (méth) acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth) acrylate de stéaryle ou leurs mélanges.

Une famille de polymères siliconés à squelette polysiloxanique greffé par des monomères organiques non siliconés convenant particulièrement bien à la mise en oeuvre de la présente invention est constituée par les polymères siliconés comportant dans leur structure le motif de formule (IV) suivant :

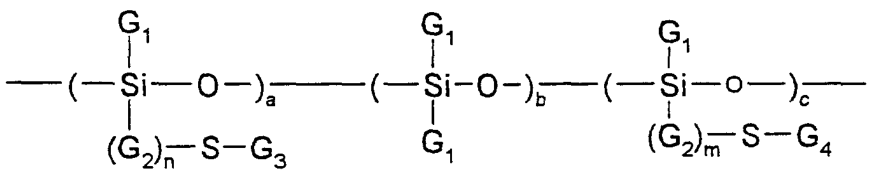

(IV)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent représente un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes :

- les radicaux $G_1$ désignent un radical alkyle, de préférence le radical méthyle ;
- n est non nul, et les radicaux $G_2$ représentent un radical divalent en $C_1$-$C_3$, de préférence un radical propylène ;
- $G_3$ représente un radical polymérique résultant de l'(homo)polymérisation d'au moins un monomère du type acide carboxylique à insaturation éthylénique, de préférence l'acide acrylique et/ou l'acide méthacrylique ;
- $G_4$ représente un radical polymérique résultant de l'(homo)polymérisation d'au moins un monomère du type (méth) acrylate d'alkyle en $C_1$-$C_{10}$, de préférence le (méth)acrylate d'isobutyle ou de méthyle.

Des exemples de polymères siliconés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle.

D'autres exemples de polymères siliconés répondant à la formule (IV) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

Les polymères siliconés greffés conformes à l'invention sont utilisés de préférence en une quantité allant de 0,01 à 20% en poids du poids total de la composition. Plus préférentiellement, cette quantité varie de 0,1 à 15% en poids et encore plus particulièrement de 0,5 à 10 % en poids.

Les copolymères blocs ayant un bloc linéaire polysiloxane-polyoxyalkylène comme unités répétitives utilisés dans le cadre de la présente invention ont de préférence la formule générale suivante :

$$([\ Y\ (R_2SiO)_a\ R'_2SiYO][(C_nH_{2n}O)_b])_c \qquad (V)$$

dans laquelle :

- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5, de préférence compris entre 5 et 200 et encore plus particulièrement entre 5 et 100.
- b est un nombre entier supérieur ou égal à 4, de préférence compris entre 4 et 200 et encore plus particulièrement entre 5 et 100.
- c est un nombre entier supérieur ou égal à 4, de préférence compris entre 4 et 1000 et encore plus particulièrement entre 5 et 300.
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 95% environ en poids du copolymère bloc,
- le poids moléculaire moyen du copolymère bloc étant d'au moins 3.000 et de préférence compris entre 5000 et 1000000 et encore plus particulièrement entre 10000 et 200000.

R et R' sont préférentiellement choisis parmi le groupe comprenant les radicaux alkyls comme par exemple les radicaux méthyle, éthyle, propyle, butyle, pentyle, hexyle, octyle, décyle, dodécyle, les radicaux aryls comme par exemple phényle, naphtyle, les radicaux aralkyls comme par exemple benzyle, phényléthyle, les radicaux tolyle, xylyle et cyclohexyle.

Y est de préférence -R''-, -R''-CO-, -R''-NHCO-, -R''-NH-CO-NH-R'''-NHCO, -R''-OCONH-R'''-NHCO-, où R'' est un groupe alkylène divalent comme par exemple l'éthylène, le propylène ou le butylène et R''' est un groupe alkylène divalent ou un groupe arylène divalent comme $-C_6H_4-$, $-C_6H_4-C_6H_4-$, $-C_6H_4-CH_2-C_6H_4-$, $-C_6H_4-C(CH_3)_2-C_6H_4-$.

Encore plus préférentiellement, Y représente un radical alkylène divalent, plus particulièrement le radical $-CH_2-CH_2-CH_2-$ ou le radical $C_4H_8$.

La préparation des copolymères blocs mis en oeuvre dans le cadre de la présente invention est décrite dans la demande européenne EP 0 492 657 A1, dont l'enseignement est inclus à titre de référence dans la présente description.

Les copolymères blocs linéaires polysiloxane-polyoxyalkylène préférés selon l'invention sont choisis parmi ceux de formule:

$$[C_4H_8O(C_nH_{2n}O)_b\ (C_mH_{2m}O)_d - C_4H_8 - SiMe_2O\ (SiMe_2O)_aSiMe_2]_c \qquad (VI)$$

ou Me représente méthyle, n et m sont des entiers allant de 2 à 4, a est un entier supérieur ou égal à 4, de préférence compris entre 5 et 200, b et d sont des entiers supérieurs ou égaux à 0, de préférence compris entre 4 et 200, b + d est supérieur ou égal à 4, de préférence compris entre 4 et 200 et c est un nombre supérieur ou égal à 4, de préférence compris entre 4 et 1000.

Parmi ces copolymères, on utilise plus particulièrement ceux ayant un motif récurrent de formule :

$$[-\ (SiMe_2O)_xSiMe_2 - C_4H_8O - (C_2H_4O)_y - (C_3H_6O)_z - C_4H_8\ -] \qquad (VII)$$

où x est un nombre compris entre 5 et 15 (bornes incluses), y est un nombre compris entre 15 et 30 (bornes incluses) ; et z est un nombre compris entre 20 et 40 (bornes incluses).

Parmi ces polymères, on utilise plus particulièrement ceux dont le rapport en poids siloxane/polyoxyalkylène est d'environ 20 et le rapport en poids polyoxyéthylène/ polyoxypropylène est d'environ 65/35.

On peut également choisir des polymères dont l'unité répétitive est de formule (VI) et dont le rapport en poids siloxane / polyoxyalkylène est d'environ 75 et le rapport en poids polyoxyéthylène/polyoxypropylène est d'environ 50/50, des polymères dont le rapport en poids siloxane/polyoxyalkylène est d'environ 35 et le rapport en poids polyoxyéthylène/polyoxypropylène est d'environ 100/0, des polymères dont le rapport en poids siloxane/polyoxyalkylène est d'environ 30 et le rapport en poids polyoxyéthylène/polyoxypropylène est d'environ 0/100.

Selon un mode de réalisation particulier de l'invention le copolymère bloc est choisi parmi les copolymères suivants :

$$[[(CH_3)_2SiO]_{41}(CH_3)_2SiCH_2CH(CH_3)CH_2\text{-}O(C_2H_4O)_{18}\text{-}(C_3H_6O)_{33}CH_2CH(CH_3)CH_2]_{16.1}$$

$$[[(CH_3)_2SiO]_{31}(CH_3)_2SiCH_2CH(CH_3)CH_2\text{-}O(C_2H_4O)_{20}\text{-}(C_3H_6O)_{29}CH_2CH(CH_3)CH_2]_{13.3}$$

$$[[(CH_3)_2SiO]_{9}(CH_3)_2SiCH_2CH(CH_3)CH_2\text{-}O(C_2H_4O)_{20}\text{-}(C_3H_6O)_{29}CH_2CH(CH_3)CH_2]_{26.3}$$

$$[[(CH_3)_2SiO]_{16}(CH_3)_2SiCH_2CH(CH_3)CH_2\text{-}O(C_2H_4O)_{18}\text{-}(C_3H_6O)_{20}CH_2CH(CH_3)CH_2]_{21.5}$$

$$[[(CH_3)_2SiO]_{9}(CH_3)_2SiCH_2CH(CH_3)CH_2\text{-}O(C_2H_4O)_{5}\text{-}CH_2CH(CH_3)CH_2]_{4.8}$$

Le copolymère bloc linéaire est utilisé de préférence en une quantité comprise entre 0,01 et 20% en poids du poids total de la composition. Plus préférentiellement, cette quantité est comprise entre 0,1 et 15% en poids et encore plus particulièrement entre 0,5 et 10 % en poids.

Selon un mode préféré de réalisation de l'invention, la composition contient en outre un polymère, de préférence anionique ou amphotère non siliconé sous forme solubilisée ou sous forme dispersée.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit milieu cosmétiquement acceptable ou utilisés sous forme de dispersion aqueuse de particules.

La composition de l'invention peut également contenir au moins un additif choisi parmi les épaississants, les esters d'acides gras, les esters d'acides gras et de glycérol, les silicones, les tensioactifs, les parfums, les conservateurs, les filtres solaires, les protéines, les vitamines, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Les compositions selon l'invention sont utilisées comme produits rincés ou comme produits non-rincés notamment pour le lavage, le soin, le conditionnement, le maintien de la coiffure ou la mise en forme des matières kératiniques telles que les cheveux.

Elles sont plus particulièrement des produits de coiffage tels que des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement des matières kératiniques telles que les cheveux consistant à appliquer sur ceux-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits. Dans tout ce qui suit, MA signifie Matière Active.

***EXEMPLE 1***

On a préparé un spray de soin capillaire conditionné en flacon pompe de composition suivante:

- Polymère siliconé greffé de formule (IV) de structure polyméthyl/méthylsiloxane à groupements propyl thio-3 polyméthacrylate d'isobutyle en solution dans une silicone volatile cyclique 4 gMA
- Copolymère bloc de formule: 1 g

$$[[(CH_3)_2SiO]_x(CH_3)_2SiCH_2CH(CH_3)CH_2O(C_2H_4O)_y\text{-}(C_3H_6O)_zCH_2CH(CH_3)CH_2]_n$$

  avec x =9 à 11, y=18 à 25, z=28 à 35 et n est tel que la viscosité d'une solution de polymère à 10 % dans l'éthanol soit d'environ 50 mPa.s.
- Ethanol qsp 100 g

***EXEMPLE 2*** Spray de coiffage en aérosol

- Polymère siliconé greffé de formule (IV) de structure polyméthyl/méthylsiloxane à groupements propyl thio-3 acide polyméthacrylique et groupements propyl thio-3 polyméthacrylate de méthyle 5 g
- Copolymère bloc de formule: 1,25 g

$$[[(CH_3)_2SiO]_x(CH_3)_2SiCH_2CH(CH_3)CH_2O(C_2H_4O)_y\text{-}(C_3H_6O)_zCH_2CH(CH_3)CH_2]_n$$

  avec x =9 à 11, y=18 à 25, z=28 à 35 et n est tel que la viscosité d'une solution de polymère à 10 % dans l'éthanol soit d'environ 50 mPa.s.
- Copolymère acétate de vinyle/acide crotonique/tertio-butyl-4-benzoate de vinyle (65/10/25) tel que décrit et préparé dans le brevet FR 2.697.160 2,5 g
- Aminométhylpropanol pour neutraliser à 100%
  le polymère siliconé greffé et le copolymère non siliconé qs
- Ethanol qsp 100 g

Schéma de pressurisation :

- Composition ci-dessus : 80g
- Mélange ternaire de n-butane, isobutane et propane (23/55/22), vendu sous la dénomination "AEROGAZ 3,2 N par la société ELF AQUITAINE 5 g
- 1,1-difluoroéthane ( SOLKANE 152 A de SOLVAY ) 15 g

***EXEMPLE 3***

On a préparé une mousse de soin capillaire de composition suivante :

- Polymère siliconé greffé de formule (IV) de structure polyméthyl/méthylsiloxane à groupements propyl thio-3 acide polyméthacrylique et groupements propyl thio-3 polyméthacrylate de méthyle 3,3 g
- Aminométhylpropanol neutralisation à 100% dudit polymère siliconé qsp
- Copolymère bloc de formule: 2,2 g

$$[[(CH_3)_2SiO]_x(CH_3)_2SiCH_2CH(CH_3)CH_2O(C_2H_4O)_y\text{-}(C_3H_6O)_zCH_2CH(CH_3)CH_2]_n$$

  avec x =9 à 11, y=18 à 25, z=28 à 35 et n est tel que la viscosité d'une solution de polymère à 10 % dans l'éthanol soit d'environ 50 mPa.s.
- Ethanol 9,7 g
- Eau déminéralisée qsp 100 g

Schéma de pressurisation :

Composition ci-dessus : 90 g

- Mélange ternaire de n-butane, isobutane et propane (23/55/22), vendu sous la dénomination "AEROGAZ 3,2 N par la société ELF AQUITAINE 10 g

Appliquée sur cheveux mouillés, cette mousse fond rapidement dans les cheveux et améliore le démêlage des cheveux mouillés. Les cheveux séchés obtenus sont nerveux et doux.

***EXEMPLE 4***

On a préparé un gel de coiffage de composition suivante :

- Copolymère constitué par : 8 g

  a) 60% en poids d'acrylate de tertiobutyle ;
  b) 20% en poids d'acide acrylique ;
  c) 20% en poids de macromère siliconé de formule :

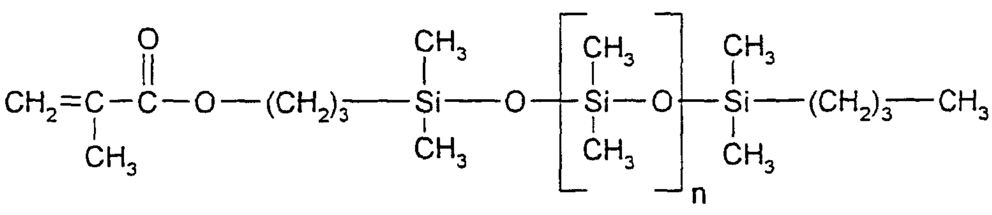

  avec n étant un nombre choisi de telle sorte que le poids moléculaire moyen en nombre du polymère soit de l'ordre de 9000-12000 ; les pourcentages en poids étant calculés par rapport au poids total des monomères.
- Aminométhylpropanol neutralisation à 100% dudit polymère siliconé qsp
- Copolymère bloc de formule: 2 g

$$[[(CH_3)_2SiO]_x(CH_3)_2SiCH_2CH(CH_3)CH_2O(C_2H_4O)_y\text{-}(C_3H_6O)_zCH_2CH(CH_3)CH_2]_n$$

  avec x =9 à 11, y=18 à 25, z=28 à 35 et n est tel que la viscosité d'une solution de polymère à 10 % dans l'éthanol soit d'environ 50 mPa.s.
- Eau déminéralisée 40 g
- Ethanol qsp 100 g

***EXEMPLE 5***

On a préparé un shampooing de composition suivante:

- Polymère siliconé greffé de formule (IV) de structure polyméthyl/méthylsiloxane à groupements propyl thio-3 acide polyméthacrylique et groupements propyl thio-3 polyméthacrylate de méthyle 2 g- Lauryl ($C_{12}/C_{14}$ 70/30) éther sulfate de sodium oxyéthyléné à 2,2 moles d'oxyde d'éthylène en solution aqueuse à 28 % de MA vendu sous le nom d'Empicol ESB 3/FL par la société Albright et Wilson 12 g MA
- Cocoylbétaïne en solution aqueuse à 28 % de MA 2 g MA
- Copolymère bloc de formule: 0,5 g

$$[[(CH_3)_2SiO]_x(CH_3)_2SiCH_2CH(CH_3)CH_2O(C_2H_4O)_y\text{-}(C_3H_6O)_zCH_2CH(CH_3)CH_2]_n$$

avec x =9 à 11, y=18 à 25, z=28 à 35 et n est tel que la viscosité d'une solution de polymère à 10 % dans l'éthanol soit d'environ 50 mPa.s.

- Parfum, séquestrant, conservateur
- Eau q.s.p. 100 g

Le pH est ajusté à 5,8 par l'addition de l'acide chlorhydrique.

***EXEMPLE 6***

On a préparé un après-shampooing à rincer de composition suivante:

- Mélange (80/20 en poids) d'alcool cétylstéarylique et d'alcool cétylstéarylique oxyéthyléné à 33 moles d'oxyde d'éthylène 2 g
- Polymère siliconé greffé de formule (IV) de structure polyméthyl/méthylsiloxane à groupements propyl thio-3 polyméthacrylate d'isobutyle en solution dans une silicone volatile cyclique 2 g
- Chlorure de béhényltriméthyl ammonium à 88 % de MA dans un mélange eau/isopropanol (15/85) vendu sous le nom de Catinal DC 80 (Toho) 2 gMA
- Copolymère bloc de formule: 0,5 g

$$[[(CH_3)_2SiO]_x(CH_3)_2SiCH_2CH(CH_3)CH_2O(C_2H_4O)_y\text{-}(C_3H_6O)_zCH_2CH(CH_3)CH_2]_n$$

avec x =9 à 11, y=18 à 25, z=28 à 35 et n est tel que la viscosité d'une solution de polymère à 10 % dans l'éthanol soit d'environ 50 mPa.s.

- Parfum, conservateur
- Eau q.s.p. 100 g

Le pH est ajusté à 5 par l'addition de l'acide chlorhydrique.

***EXEMPLES COMPARATIFS***

***I POUVOIR FIXANT***

On a étudié et comparé le pouvoir fixant obtenu par les trois formulations A, B et C suivantes :

***COMPOSITION A (art antérieur) :***

- Polymère ***$P_1$*** siliconé greffé de formule (IV) de structure polyméthyl/méthylsiloxane à groupements propyl thio-3 acide polyméthacrylique et groupements propyl thio-3 polyméthacrylate de méthyle (solution aqueuse à 10%) 5 g MA
- Monométhyléther de tripropylèneglycol (plastifiant) 0,5 g
- Aminométhylpropanol neutralisation à 100% du polymère siliconé greffé qsp

- Ethanol à 98,5% qsp 100 g

***COMPOSITION B (invention)*** **:**

- Polymère ***P₁*** siliconé greffé de formule (IV) de structure polyméthyl/méthylsiloxane à groupements propyl thio-3 acide polyméthacrylique et groupements propyl thio-3 polyméthacrylate de méthyle (solution à 10%) 5 gMA
- Copolymère **(*P2*)** bloc de formule: 1 g

$$[[(CH_3)_2SiO]_x(CH_3)_2SiCH_2CH(CH_3)CH_2O(C_2H_4O)_y\text{-}(C_3H_6O)_zCH_2CH(CH_3)CH_2]_n$$

avec x =9 à 11, y=18 à 25, z=28 à 35 et n est tel que la viscosité d'une solution de polymère à 10 % dans l'éthanol soit d'environ 50 mPa.s.

- Monométhyléther de tripropylèneglycol (plastifiant) 0,5 g
- Aminométhylpropanol neutralisation à 100% du polymère siliconé greffé qsp
- Ethanol à 98,5% qsp 100 g

***COMPOSITION C (art antérieur)*** **:**

- Polymère ***P₁*** siliconé greffé de formule (IV) de structure polyméthyl/méthylsiloxane à groupements propyl thio-3 acide polyméthacrylique et groupements propyl thio-3 polyméthacrylate de méthyle (solution aqueuse à 10%) 5 g MA
- Aminométhylpropanol neutralisation à 100% du polymère siliconé greffé qsp
- Monométhyléther de tripropylèneglycol (plastifiant) 0,5 g
- Diméthiconecopolyol vendu sous la dénomination MIRASIL DMCO par la société RHONE POULENC 1g
- Ethanol à 98,5% qsp 100 g

***MODE OPERATOIRE***

On effectue un test de mesure de la force des liaisons formées entre les cheveux par une laque capillaire selon les principes de la méthode décrite dans l'article de R.RANDALL WICKETT, JOHN A. SRAMEK et CYNTHIA M. TROBAUGH dans J. Soc. Cosmet. Chem. 43, 169-178 (Mai/Juin 1992).
Pour chaque formulation testée, on prend un cheveu unitaire. On effectue sur le cheveu une simple boucle ayant un diamètre de 2 cm approximativement, au moyen d'un support cylindrique. On trempe le cheveu ainsi bouclé dans la formulation et on le laisse sécher sous atmosphère conditionnée (20°C et 50% d'humidité). On coupe la boucle fixée par la formulation A, B ou C. On obtient ainsi 2 demi-cheveux liés entre eux par un point de fixation.
On fixe les extrémités, situées de part et d'autre du point de fixation, à chacune des 2 mâchoires d'un appareil du type INSTRON® mesurant la force de tension en Newtons exercée sur les demi-cheveux.
On mesure la force moyenne (sur dix essais) $F_A$, $F_B$ et $F_C$ (spécifique à la composition A, B ou C) nécessaire pour rompre le point de fixation reliant les deux demi-cheveux et formé par la formulation A, B ou C.
On détermine l'amélioration apportée par l'association du polymère greffé siliconé $P_1$ et du copolymère $P_2$ par rapport au polymère $P_1$ (Composition A) utilisé seul ou par rapport à l'association de $P_1$ et d'une silicone polyoxyalkylénée (Composition C) en calculant la variation relative de la force de rupture exprimée en pourcentage mesurée selon la formule suivante :

$$(F_B - F_A / F_A) \times 100$$

ou

$$(F_B - F_C / F_C) \times 100$$

Les résultats sont indiqués dans le tableau ci-après :

| FORMULATION TESTEE | FORCE MOYENNE DE RUPTURE EXPRIMEE EN NEWTONS | AMELIORATION EN % DU POUVOIR FIXANT |
|---|---|---|
| B (Invention) | 0,24 | - |
| A (Comparatif) | 0,09 | 166 % |
| C (Comparatif) | 0,10 | 140% |

Le pouvoir fixant de la composition selon l'invention est nettement supérieur à celui des compositions de l'art antérieur.

**Revendications**

1. Composition cosmétique ou dermatologique destinée au traitement des matières kératiniques, **caractérisée par le fait qu'**elle contient dans un milieu cosmétiquement ou dermatologiquement acceptable au moins un polymère siliconé greffé comprenant une portion polysiloxane et une portion constituée d'une chaîne organique non-siliconée, l'une des deux portions constituant la chaîne principale du polymère l'autre étant greffée sur la dite chaîne principale et au moins un copolymère ayant comme unités répétitives un bloc linéaire polysiloxane-polyoxyalkylène.

2. Composition selon la revendication 1, **caractérisée par le fait que** le polymère siliconé greffé est choisi dans le groupe constitué par les polymères à squelette organique non siliconé greffé par des monomères contenant un polysiloxane, les polymères à squelette polysiloxanique greffé par des monomères organiques non siliconés et leurs mélanges.

3. Composition selon la revendication 1 ou 2, **caractérisée par le fait qu'**elle contient au moins un polymère siliconé greffé, à squelette organique non siliconé, constitué d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

4. Composition selon la revendication 3, **caractérisée par le fait que** les monomères organiques non siliconés constituant la chaîne principale du polymère siliconé greffé sont choisis dans le groupe constitué par des monomères à insaturation éthylénique polymérisables par voie radicalaire, des monomères polymérisables par polycondensation, des monomères à ouverture de cycle.

5. Composition selon l'une quelconque des revendications 1 à 4, **caractérisée par le fait qu'**elle contient au moins un copolymère greffé siliconé comprenant :

   a) de 0 à 98% en poids d'au moins un monomère (A) lipophile de faible polarité à insaturation éthylénique de faible polarité, polymérisable par voie radicalaire ;
   b) de 0 à 98% en poids d'au moins un monomère (B) hydrophile polaire à insaturation éthylénique, copolymérisable avec le ou les monomères du type (A) ;
   c) de 0,01 à 50 % en poids d'au moins un macromère polysiloxane (C) de formule générale :

$$X(Y)_nSi(R)_{3-m}\,Z_m \qquad (I)$$

   où :

   X désigne un groupe vinylique copolymérisable avec les monomères (A) et (B) ;
   Y désigne un groupe de liaison divalent ;
   R désigne un hydrogène, un alkyle ou un alcoxy en $C_1$-$C_6$, un aryle $C_6$-$C_{12}$ ;
   Z désigne un motif polysiloxane monovalent ayant un poids moléculaire moyen en nombre d'au moins 500 ;
   n est 0 ou 1 et m est un entier allant de 1 à 3 ; les pourcentages étant calculés par rapport au poids total des monomères (A), (B) et (C).

**6.** Composition selon la revendication 5, **caractérisée par le fait que** les monomères lipophiles (A) sont choisis dans le groupe constitué par les esters d'acide acrylique ou méthacrylique d'alcools en $C_1$-$C_{18}$ ; le styrène ; les macromères polystyrène ; l'acétate de vinyle ; le propionate de vinyle ; l'alpha-méthylstyrène ; le tertio-butylstyrène ; le butadiène ; le cyclohexadiène ; le cyclohexadiène ; l'éthylène ; le propylène ; le vinyltoluène ; les esters d'acide acrylique ou méthacrylique et de 1,1-dihydroperfluoroalcanols ou de leurs homologues ; les esters d'acide acrylique ou méthacrylique et de oméga-hydrydofluroalcanols ; les esters d'acide acrylique ou méthacrylique et de fluoroalkylsulfoamido-alcools; les esters d'acide acrylique ou méthacrylique et d'alcools fluoroalkyliques ; les esters d'acide acrylique ou méthacrylique et de fluroéthers d'alcools ; ou leurs mélanges.

**7.** Composition selon la revendication 6, **caractérisée par le fait que** les monomères lipophiles (A) sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-butylperflurooctane sulfonamido)-éthylacrylate, le 2-(N-méthylperflurooctane sulfonamido)-éthylacrylate et leurs mélanges.

**8.** Composition selon l'une quelconque des revendications 5 à 7, **caractérisée par le fait que** les monomères polaires (B) sont choisis dans le groupe constitué par l'acide acrylique, l'acide méthacrylique, le N,N-diméthylacrylamide, le méthacrylate de diméthyl-aminoéthyle, le méthacrylate de diméthylaminoéthyle quatemisé, le (méth) acrylamide, le N-t-butylacrylamide, l'acide maléique, l'anhydride maléique et leurs demi-esters, les (méth)acrylates hydroxyalkylés, le chlorure de diallyldiméthylammonium, la vinyl-pyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinyl caprolactame ou leurs mélanges.

**9.** Composition selon la revendication 8, **caractérisée par le fait que** les monomères polaires (B) sont choisis dans le groupe constitué par l'acide acrylique, le N,N-diméthylacrylamide, le méthacrylate de diméthylaminoéthyle, le méthacrylate de diméthylaminoéthyle quatemisé, la vinylpyrrolidone et leurs mélanges.

**10.** Composition selon l'une quelconque des revendications 5 à 9, **caractérisée par le fait que** le macromère polysiloxane (C) correspond à la formule générale suivante (II) :

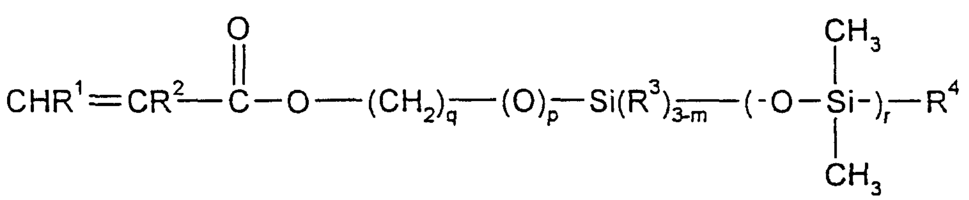

(II)

- dans laquelle :

$R^1$ est hydrogène ou -COOH ;
$R^2$ est hydrogène, méthyle ou $-CH_2COOH$;
$R^3$ est alkyle, alcoxy ou alkylamino en $C_1$-$C_6$, aryle en $C_6$-$C_{12}$ ou hydroxyle ;
$R^4$ est alkyle, alcoxy ou alkylamino en $C_1$-$C_6$, aryle en $C_6$-$C_{12}$ ou hydroxyle ;
q est un entier de 2 à 6 ;
p est 0 ou 1 ;
r est un nombre entier de 5 à 700 ;
m est un entier allant de 1 à 3.

**11.** Composition selon l'une quelconque des revendications 5 à 10, **caractérisée par le fait que** le macromère polysiloxane (C) correspond à la formule générale suivante :

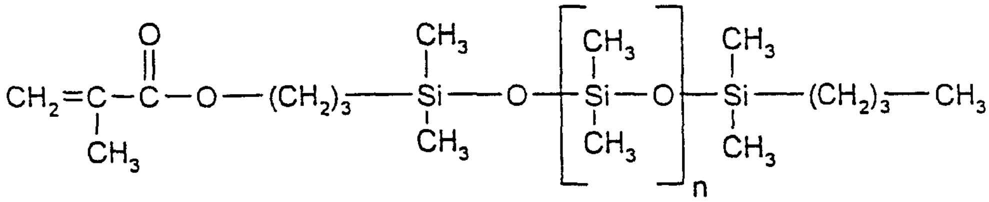

avec n étant un nombre allant de 5 à 700.

**12.** Composition selon l'une quelconque des revendications 1 à 11, **caractérisée par le fait qu'**elle contient au moins un copolymère susceptible d'être obtenu par polymérisation radicalaire à partir du mélange de monomères constitué par :

a) 60% en poids d'acrylate de tertiobutyle ;
b) 20% en poids d'acide acrylique ;
c) 20% en poids de macromère siliconé de formule :

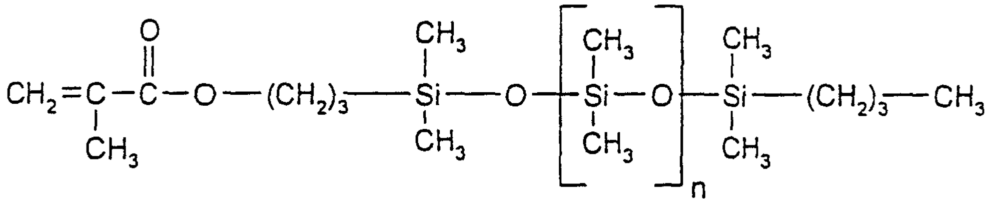

avec n étant un nombre allant de 5 à 700 ; les pourcentages en poids étant calculés par rapport au poids total des monomères.

**13.** Composition selon l'une quelconque des revendications 1 à à 11, **caractérisée par le fait qu'**elle contient au moins un copolymère susceptible d'être obtenu par polymérisation radicalaire à partir du mélange de monomères constitué par :

a) 80% en poids d'acrylate de tertiobutyle ;
b) 20% en poids de macromère siliconé de formule :

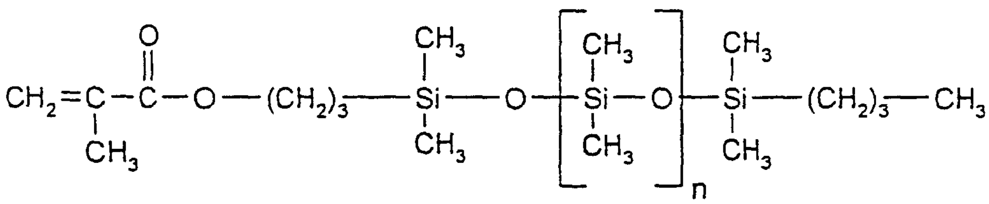

avec n étant un nombre allant de 5 à 700 ; les pourcentages en poids étant calculés par rapport au poids total des monomères.

**14.** Composition selon l'une quelconque des revendications 5 à 13, **caractérisée par le fait que** le polymère à squelette organique non siliconé greffé par des monomères contenant un polysiloxane a un poids moléculaire moyen en nombre allant de 10.000 à 2.000.000 et une température de transition vitreuse Tg ou une température de fusion cristalline Tm d'au moins -20°C.

**15.** Composition selon l'une quelconque des revendications 1 à 4, **caractérisée par le fait qu'**elle contient au moins un polymère à squelette organique non siliconé greffé par des monomères contenant un polysiloxane, susceptible d'être obtenu par extrusion réactive d'un macromère polysiloxane ayant une fonction réactive terminale sur un polymère du type polyoléfine comportant des groupes réactifs susceptibles de réagir avec la fonction réactive terminale du macromère polysiloxane pour former une liaison covalente permettant le greffage de la silicone sur la chaîne principale de la polyoléfine.

**16.** Composition selon la revendication 15, **caractérisée par le fait que** la polyoléfine réactive est choisie dans le groupe constitué par les polyéthylènes ou les polymères de monomères dérivés de l'éthylène comportant des fonctions réactives susceptibles de réagir avec la fonction terminale du macromère polysiloxane.

**17.** Composition selon la revendication 15 ou 16, **caractérisée par le fait que** la polyoléfine réactive est choisie dans le groupe constitué par les copolymères d'éthylène ou de dérivés d'éthylène et de monomères choisis parmi ceux

comportant une fonction carboxylique ; ceux comportant une fonction anhydride d'acide ; ceux comportant une fonction chlorure d'acide ; ceux comportant une fonction ester ; ceux comportant une fonction isocyanate.

**18.** Composition selon l'une quelconque des revendications 15 à 17, **caractérisée par le fait que** le macromère polysiloxane est un polysiloxane comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires.

**19.** Composition selon l'une quelconque des revendications 15 à 18, **caractérisée par le fait que** le macromère polysiloxane est un polysiloxane répondant à la formule générale (III):

$$T\text{-}(CH_2)_s\text{-}Si\text{-}[(O\text{-}SiR^5R^6)_t\text{-}R^7]_y \qquad \text{(III)}$$

dans laquelle T est choisi dans le groupe constitué par $NH_2$, NHR', une fonction époxy, OH, SH ; $R^5$, $R^6$, $R^7$ et R', indépendamment, désignent un alkyle en $C_1$-$C_6$, phényle, benzyle, ou alkylphényle en $C_6$-$C_{12}$, hydrogène ; s est un nombre de 2 à 100 ; t est un nombre de 0 à 1000 et y est un nombre de 1 à 3.

**20.** Composition selon la revendication 1 ou 2, **caractérisée par le fait qu'**elle contient au moins un polymère siliconé greffé, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un groupement organique ne comportant pas de silicone.

**21.** Composition selon la revendication 20, **caractérisée par le fait que** le polymère à squelette polysiloxanique greffé par des monomères organiques non siliconés est susceptible d'être obtenu par copolymérisation radicalaire entre d'une part au moins un monomère organique anionique non siliconé présentant une insaturation éthylénique et/ou un monomère organique hydrophobe non siliconé présentant une insaturation éthylénique et d'autre part un polysiloxane présentant dans sa chaîne au moins un groupement fonctionnel capable de venir réagir sur lesdites insaturations éthyléniques desdits monomères non siliconés.

**22.** Composition selon la revendication 21, **caractérisée par le fait que** le monomères organique anionique à insaturation éthylénique est choisi, seul ou sous forme de mélange de monomères, parmi les acides carboxyliques insaturés, linéaires ou ramifiés.

**23.** Composition selon la revendication 22, **caractérisée par le fait que** le monomères organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique ou leurs sels d'alcalins, d'alcalino-terreux ou d'ammonium, ou leurs mélanges.

**24.** Composition selon la revendication 22, **caractérisée par le fait que** le monomère organique hydrophobe à insaturation éthylénique est choisi, seul ou en mélange de monomères, parmi les esters d'acide acrylique d'alcanol et/ou les esters d'acide méthacrylique d'alcanol, de préférence l'alcanol étant en $C_1$-$C_{18}$.

**25.** Composition selon la revendications 24, **caractérisée par le fait que** le monomère organique hydrophobe à insaturation éthylénique est choisi, seul ou en mélange de monomères dans le groupe constitué par le (méth)acrylate d'isooctyle, le (méth) acrylate d'isononyle, le 2-éthylhexyl(méth)acrylate, le (méth)acrylate de lauryle, le (méth) acrylate d'isopentyle, le (méth)acrylate de n-butyle, le (méth)acrylate d'isobutyle, le (méth)acrylate de méthyle, le (méth)acrylate de tertio-butyle, le (méth) acrylate de tridécyle, le (méth)acrylate de stéaryle.

**26.** Composition selon l'une quelconque des revendications 20 à 25, **caractérisée par le fait que** le polymère siliconé greffé comprend sur la chaîne silicone principale, au moins un groupement organique à caractère anionique obtenu par l'(homo) polymérisation radicalaire d'au moins un monomère anionique de type acide carboxylique insaturé, partiellement ou totalement neutralisé sous la forme d'un sel.

**27.** Composition selon l'une quelconque des revendications 20 à 26, **caractérisée par le fait que** le polymère siliconé greffé est choisi parmi les polymères siliconés comportant dans leur structure le motif de formule (IV) suivant

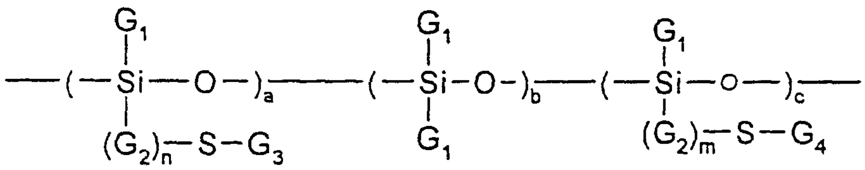

(IV)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo) polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

**28.** Composition selon la revendication 27, **caractérisée par le fait que** le motif de formule (IV) présente au moins l'une des caractéristiques suivantes:

- les radicaux $G_1$ désignent un radical alkyle en $C_1$-$C_{10}$ ;
- n est non nul, et les radicaux $G_2$ représentent un radical divalent en $C_1$-$C_3$ ;
- $G_3$ représente un radical polymérique résultant de l'(homo)polymérisation d'au moins un monomère du type acide carboxylique à insaturation éthylénique ;
- $G_4$ représente un radical polymérique résultant de l'(homo)polymérisation d'au moins un monomère du type (méth)acrylate d'alkyle en $C_1$-$C_{10}$ ;

**29.** Composition selon la revendication 27 ou 28, **caractérisée par le fait que** le motif de formule (IV) présente simultanément les caractéristiques suivantes :

- les radicaux $G_1$ désignent un radical méthyle ;
- n est non nul, et les radicaux $G_2$ représentent un radical propylène ;
- $G_3$ représente un radical polymérique résultant de l'(homo)polymérisation d'au moins l'acide acrylique et/ou l'acide méthacrylique ;
- $G_4$ représente un radical polymérique résultant de l'(homo)polymérisation d'au moins le (méth)acrylate d'isobutyle ou de méthyle.

**30.** Composition selon l'une quelconque des revendications 20 à 29, **caractérisée par le fait que** la masse moléculaire en nombre du polymère à squelette polysiloxanique greffé par des monomères organiques non siliconés varie de 10 000 à 1 000 000 environ, et encore plus préférentiellement de 10 000 à 100 000 environ.

**31.** Composition selon l'une quelconque des revendications 1 à 30, **caractérisée par le fait que** le ou les polymères siliconés greffés sont utilisés en une quantité allant de 0,01 à 20% en poids par rapport au poids total de la composition, de préférence de 0,1 à 15% en poids et plus particulièrement de 0,5 à 10 % en poids.

**32.** Composition selon l'une quelconque des revendications précédentes, **caractérisée en ce que** le copolymère bloc linéaire répond à la formule générale:

$$([\,Y\,(R_2SiO)_a\,R'_2SiYO][(C_nH_{2n}O)_b])_c$$

dans laquelle :

- R et R' identiques ou différents, représentent un radical hydrocarboné monovalent ne contenant pas d'insaturation aliphatique,
- n est un nombre entier compris entre 2 et 4,
- a est un nombre entier supérieur ou égal à 5,
- b est un nombre entier supérieur ou égal à 4,
- c est un nombre entier supérieur ou égal à 4,

- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 95% environ en poids du copolymère bloc,
- le poids moléculaire moyen du copolymère bloc étant d'au moins 3000.

**33.** Composition selon la revendication 32, **caractérisée en ce que** R et R' sont choisis dans le groupe constitué par les radicaux méthyle, éthyle, propyle, butyle, pentyle, hexyle, octyle, décyle, dodécyle, phényle, naphtyle, benzyle, phényléthyle, tolyle, xylyle et cyclohexyle.

**34.** Composition selon l'une quelconque des revendications 32 ou 33, **caractérisée en ce que** Y est choisi dans le groupe constitué par-R"-, -R"-CO-, -R"-NHCO-, R"-NH-CONH-R'"NHCO, -R"-OCONH-R'"-NHCO-, où R" représente un radical éthylène, propylène ou butylène et R'" représente un groupe $-C_6H_4-$, $-C_6H_4-C_6H_4-$, $-C_6H_4-CH_2-C_6H_4-$ ou $-C_6H_4-CH(CH_3)_2-C_6H_4-$.

**35.** Composition selon l'une quelconque des revendications précédentes, **caractérisée par le fait que** les copolymères blocs linéaires polysiloxane-polyoxyalkylène sont choisis parmi ceux de formule:

$$[C_4H_8(C_nH_{2n}O)_b - C_4H_8 - SiMe_2O\ (SiMe_2O)_aSiMe_2]_c$$

où Me désigne méthyle, n est un entier de 2 à 4, a et b sont des entiers supérieurs ou égaux à 4, c est un nombre supérieur ou égal à 4.

**36.** Composition selon la revendication 35, **caractérisée par le fait que** les copolymères blocs linéaires polysiloxane-polyoxyalkylène ont pour motif récurrent celui de structure :

$$[-\ (SiMe_2O)_xSiMe_2 - C_4H_8O-(C_2H_4O)_y - (C_3H_6O)_z - C_4H_8-]$$

où x est un nombre compris entre 5 et 15 inclus, y est un nombre compris entre 15 et 30 inclus ; et z est un nombre compris entre 20 et 40.

**37.** Composition selon l'une quelconque des revendications 1 à 35, **caractérisée en ce que** le copolymère bloc est choisi parmi le groupe comprenant les composés suivants :

$$[[(CH_3)_2SiO]_{41}(CH_3)_2SiCH_2CH(CH_3)CH_2-O(C_2H_4O)_{18}-(C_3H_6O)_{33}CH_2CH(CH_3)CH_2]_{16.1}$$

$$[[(CH_3)_2SiO]_{31}(CH_3)_2SiCH_2CH(CH_3)CH_2-O(C_2H_4O)_{20}-(C_3H_6O)_{29}CH_2CH(CH_3)CH_2]_{13.3}$$

$$[[(CH_3)_2SiO]_{9}(CH_3)_2SiCH_2CH(CH_3)CH_2-O(C_2H_4O)_{20}-(C_3H_6O)_{29}CH_2CH(CH_3)CH_2]_{26.3}$$

$$[[(CH_3)_2SiO]_{16}(CH_3)_2SiCH_2CH(CH_3)CH_2-O(C_2H_4O)_{18}-(C_3H_6O)_{20}CH_2CH(CH_3)CH_2]_{21.5}$$

$$[[(CH_3)_2SiO]_{9}(CH_3)_2SiCH_2CH(CH_3)CH_2-O(C_2H_4O)_{5}-CH_2CH(CH_3)CH_2]_{4.8}$$

**38.** Composition selon l'une quelconque des revendications précédentes, **caractérisé par le fait que** le ou les copolymères bloc linéaire sont présents dans des concentrations allant de 0,01 à 20% en poids par rapport au poids total de la composition, de préférence de 0,1 à 15% en poids et plus particulièrement de 0,5 à 10 % en poids.

**39.** Composition selon l'une quelconque des revendications précédentes, **caractérisée par le fait qu'**elle contient en plus au moins un additif choisi dans le groupe constitué par les épaississants, les esters d'acides gras, les esters

d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

**40.** Composition selon l'une quelconque des revendications précédentes, **caractérisée par le fait qu'**elle contient un polymère anionique ou amphotère non siliconé sous forme solubilisée ou sous forme dispersée.

**41.** Composition selon l'une quelconque des revendications précédentes, **caractérisée par le fait que** le milieu cosmétiquement ou dermatologiquement acceptable est constitué par de l'eau ou un mélange d'eau et d'au moins un solvant cosmétiquement acceptable.

**42.** Composition selon la revendication 41, **caractérisée par le fait que** les solvants cosmétiquement acceptables sont choisis dans le groupe constitué par les monoalcools, les polyalcools, les éthers de glycol, les esters d'acides gras et leurs mélanges.

**43.** Composition selon l'une quelconque des revendications précédentes, **caractérisée par le fait que** les matières kératiniques sont des cheveux.

**44.** Composition selon l'une quelconque des revendications précédentes, **caractérisée par le fait qu'**elle se présente sous forme de gel, de lait, de crème, de lotion plus ou moins épaissie ou de mousse.

**45.** Composition selon l'une quelconque des revendications précédentes, **caractérisée par le fait qu'**elle est un produit de coiffage.

**46.** Composition selon l'une quelconque des revendications précédentes, **caractérisée par le fait qu'**elle est un produit capillaire choisi dans le groupe constitué par des shampooings, des produits capillaires à rincer ou non, à appliquer avant ou après un shampooing, une coloration, une décoloration, une permanente ou un défrisage.

**47.** Composition selon l'une quelconque des revendications précédentes, **caractérisée par le fait qu'**elle est conditionnée sous forme de vaporisateur, de flacon pompe ou bien dans un récipient aérosol en vue d'obtenir un spray, une laque ou une mousse.

**48.** Composition selon l'une quelconque des revendications précédentes, **caractérisée par le fait que** le polymère siliconé greffé est dissous dans le milieu cosmétiquement ou dermatologiquement acceptable ou utilisé sous forme de dispersion aqueuse de particules.

**49.** Procédé non-thérapeutique de traitement des matières kératiniques en particulier des cheveux humains, **caractérisée par le fait qu'**il consiste à appliquer sur lesdites matières une composition telle que définie selon l'une quelconque des revendications précédentes puis à effectuer éventuellement un rinçage à l'eau.

## Patentansprüche

**1.** Kosmetische oder dermatologische Zusammensetzung, die zur Behandlung von Keratinsubstanzen vorgesehen ist, **dadurch gekennzeichnet, daß** sie in einem kosmetisch oder dermatologisch akzeptablen Medium mindestens ein gepfropftes Siliconpolymer mit einem Polysiloxanbereich und einem Bereich, der aus einer nicht siliconhaltigen organischen Kette besteht, wobei ein Bereich die Hautkette des Polymers bildet und der andere auf die Hauptkette gepfropft ist, und mindestens ein Copolymer enthält, das als wiederkehrende Einheit einen geradkettigen Polysiloxan-Polyoxyalkylen-Block aufweist.

**2.** Zusammensetzung nach Anspruch 1, **dadurch gekennzeichnet, daß** das gepfropfte Siliconpolymer unter den Polymeren, die ein nicht siliconhaltiges, organisches Grundgerüst aufweisen, auf das polysiloxanhaltige Monomere gepfropft sind, Polymeren, die ein Polysiloxangerüst aufweisen, auf das nicht siliconhaltige, organische Monomere gepfropft sind, und deren Gemischen ausgewählt ist.

**3.** Zusammensetzung nach Anspruch 1 oder 2, **dadurch gekennzeichnet, daß** sie mindestens ein gepfropftes Siliconpolymer mit einem nicht siliconhaltigen, organischen Grundgerüst enthält, das aus einer organischen Hauptkette besteht, die aus organischen Monomeren gebildet wird, die kein Silicon enthalten, auf die in der Kette sowie

gegebenenfalls an mindestens einem ihrer Enden ein Polysiloxanmakromer gepfropft ist.

**4.** Zusammensetzung nach Anspruch 3, **dadurch gekennzeichnet, daß** die nicht siliconhaltigen, organischen Monomere, die die Hauptkette des gepfropften Siliconpolymers bilden, unter den auf radikalischem Wege polymerisierbaren Monomeren mit ethylenischer Doppelbindung, den durch Polykondensation polymerisierbaren Monomeren und den unter Ringöffnung polymerisierbaren Monomeren ausgewählt sind.

**5.** Zusammensetzung nach einem der Ansprüche 1 bis 4, **dadurch gekennzeichnet, daß** sie mindestens ein gepfropftes Siliconpolymer enthält, das aufweist:

a) 0 bis 98 Gew.-% mindestens eines lipophilen Monomers (A) von geringer Polarität mit ethylenischer Doppelbindung, das radikalisch polymerisierbar ist;
b) 0 bis 98 Gew.-% mindestens eines polaren hydrophilen Monomers (B) mit ethylenischer Doppelbindung, das mit dem (den) Monomer(en) vom Typ (A) copolymerisierbar ist;
c) 0,01 bis 50 Gew.-% mindestens eines Polysiloxanmakromers (C) der allgemeinen Formel:

$$X(Y)_nSi(R)_{3-m}Z_m \quad (I)$$

worin bedeuten:

- X eine mit den Monomeren (A) und (B) copolymerisierbare Vinylgruppe;
- Y eine zweiwertige Verbindungsgruppe;
- R Wasserstoff, Alkyl oder Alkoxy mit 1 bis 6 Kohlenstoffatomen, $C_{6-12}$-Aryl;
- Z eine einwertige Polysiloxaneinheit mit einem Zahlenmittel des Molekulargewichts von mindestens 500;
- n 0 oder 1 und m eine ganze Zahl von 1 bis 3; wobei die Prozentzahlen bezogen auf das Gesamtgewicht der Monomeren (A), (B) und (C) angegeben sind.

**6.** Zusammensetzung nach Anspruch 5, **dadurch gekennzeichnet, daß** die lipophilen Monomere (A) unter Estern von Acrylsäure oder Methacrylsäure und $C_{1-18}$-Alkoholen; Styrol; Polystyrolmakromeren; Vinylacetat; Vinylpropionat; $\alpha$-Methylstyrol; tert.-Butylstyrol; Butadien; Cyclohexadien; Ethylen; Propylen; Vinyltoluol; Estern von Acrylsäure oder Methacrylsäure und 1,1-Dihydroperfluoralkanolen oder ihren homologen Verbindungen; Estern von Acrylsäure oder Methacrylsäure und omega-Hydridofluoralkanolen; Estern von Acrylsäure oder Methacrylsäure und Fluoralkylsulfonamidoalkoholen; Estern von Acrylsäure oder Methacrylsäure und Fluoralkylalkoholen; Estern von Acrylsäure oder Methacrylsäure und Fluorethern von Alkoholen; oder deren Gemischen ausgewählt sind.

**7.** Zusammensetzung nach Anspruch 6, **dadurch gekennzeichnet, daß** die lipophilen Monomere (A) unter n-Butylmethacrylat, Isobutylmethacrylat, tert.-Butylacrylat, tert.-Butylmethacrylat, 2-Ethylhexylmethacrylat, Methylmethacrylat, 2-(N-Butyl-perfluoroctansulfonamido)-ethylacrylat; 2-(N-Methyl-perfluoroctan-sulfonamido)-ethylacrylat oder deren Gemischen ausgewählt sind.

**8.** Zusammensetzung nach einem der Ansprüche 5 bis 7, **dadurch gekennzeichnet, daß** die polaren Monomere (B) unter Acrylsäure, Methacrylsäure, N,N-Dimethylacrylamid, Dimethylaminoethylmethacrylat, quaternisiertem Dimethylaminoethylmethacrylat, (Meth)acrylamid, N-t-Butyl-acrylamid, Maleinsäure, Maleinsäureanhydrid und seinen Halbestern, hydroxyalkylierten (Meth)acrylaten, Diallyldimethylammoniumchlorid, Vinylpyrrolidon, Vinylethern, Maleimiden, Vinylpyridin, Vinylimidazol, heterocyclischen polaren Vinylverbindungen, Styrolsulfonat, Allylalkohol, Vinylalkohol, Vinylcaprolactam oder deren Gemischen ausgewählt sind.

**9.** Zusammensetzung nach Anspruch 8, **dadurch gekennzeichnet, daß** die polaren Monomere (B) unter Acrylsäure, N,N-Dimethylacrylamid, Dimethylaminoethylmethacrylat, quaternisiertem Dimethylaminoethylmethacrylat, Vinylpyrrolidon und deren Gemischen ausgewählt sind.

**10.** Zusammensetzung nach einem der Ansprüche 5 bis 9, **dadurch gekennzeichnet, daß** das Polysiloxanmakromer (C) unter den Verbindungen der folgenden allgemeinen Formel (II) ausgewählt ist:

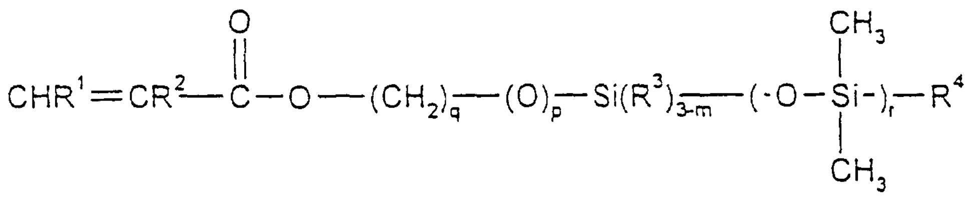 (II),

worin bedeuten:

$R^1$ Wasserstoff oder -COOH,
$R^2$ Wasserstoff, Methyl oder $-CH_2COOH$,
$R^3$ Alkyl, Alkoxy oder Alkylamino mit 1 bis 6 Kohlenstoffatomen, $C_{6-12}$-Aryl oder Hydroxy,
$R^4$ Alkyl, Alkoxy oder Alkylamino mit 1 bis 6 Kohlenstoffatomen, $C_{6-12}$-Aryl oder Hydroxy,
q eine ganze Zahl von 2 bis 6,
p 0 oder 1,
r eine ganze Zahl von 5 bis 700,
m eine ganze Zahl von 1 bis 3.

**11.** Zusammensetzung nach einem der Ansprüche 5 bis 10, **dadurch gekennzeichnet, daß** das Polysiloxanmakromer (C) der folgenden allgemeinen Formel entspricht:

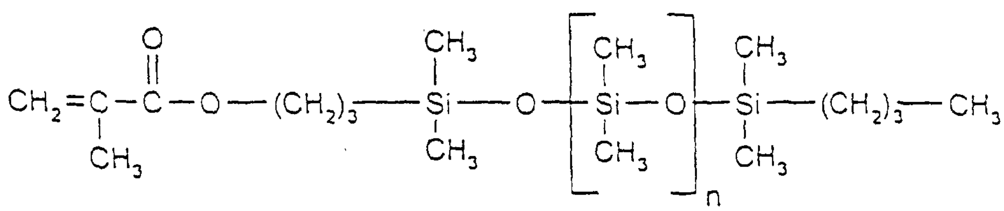

wobei n eine Zahl im Bereich von 5 bis 700 bedeutet.

**12.** Zusammensetzung nach einem der Ansprüche 1 bis 11, **dadurch gekennzeichnet, daß** sie mindestens ein Copolymer enthält, das durch radikalische Polymerisation ausgehend von folgendem Monomerengemisch erhältlich ist:

a. 60 Gew.-% tert.-Butylacrylat,
b. 20 Gew.-% Acrylsäure,
c. 20 Gew.-% Siliconmakromer der Formel:

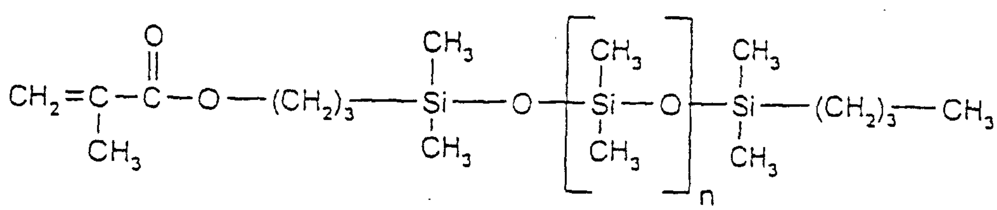

wobei n eine Zahl von 5 bis 700 ist; die Prozentzahlen sind bezogen auf das Gesamtgewicht der Monomere angegeben.

**13.** Zusammensetzung nach einem der Ansprüche 1 bis 11, **dadurch gekennzeichnet, daß** sie mindestens ein Copolymer enthält, das durch radikalische Polymerisation ausgehend von folgendem Monomerengemisch erhältlich ist:

a) 80 Gew.-% tert.-Butylacrylat,

b) 20 Gew.-% Siliconmakromer der Formel:

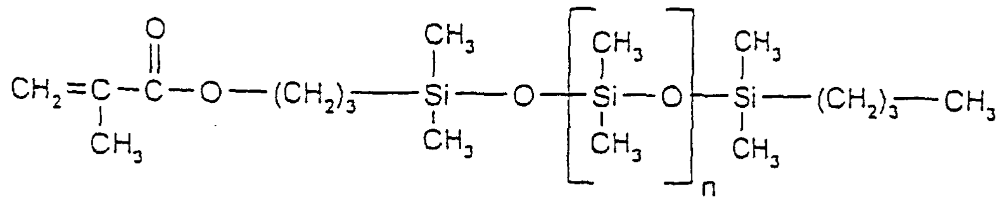

wobei n eine Zahl von 5 bis 700 ist; die Prozentzahlen sind bezogen auf das Gesamtgewicht der Monomere angegeben.

**14.** Zusammensetzung nach einem der Ansprüche 5 bis 13, **dadurch gekennzeichnet, daß** das Polymer mit einem nicht siliconhaltigen, organischen Grundgerüst, das mit polysiloxanhaltigen Monomeren gepfropft ist, ein Zahlenmittel des Molekulargewichts von 10 000 bis 2 000 000 und eine Glasübergangstemperatur Tg oder eine kristalline Schmelztemperatur $T_m$ von mindestens -20 °C aufweist.

**15.** Zusammensetzung nach einem der Ansprüche 1 bis 4, **dadurch gekennzeichnet, daß** sie mindestens ein Polymer mit einem nicht siliconhaltigen, organischen Grundgerüst enthält, das mit polysiloxanhaltigen Monomeren gepfropft ist, das durch reaktive Extrusion eines Polysiloxanmakromers mit einer reaktiven Endgruppe und eines Polymers vom Polyolefintyp erhältlich ist, das Gruppen aufweist, die mit der reaktiven Endgruppe des Polysiloxanmakromers reagieren können, wodurch eine kovalente Bindung ausgebildet wird, über die das Silicon auf die Polyolefin-Hauptkette gepfropft werden kann.

**16.** Zusammensetzung nach Anspruch 15, **dadurch gekennzeichnet, daß** das reaktive Polyolefin unter den Polyethylenen oder den Polymeren von Monomeren, die von Ethylen abgeleitet sind, ausgewählt sind, die reaktive Gruppen aufweisen, die mit der Endgruppe des Polysiloxanmakromers reagieren können.

**17.** Zusammensetzung nach Anspruch 15 oder 16, **dadurch gekennzeichnet, daß** das reaktive Polyolefin unter den Ethylencopolymeren oder Derivaten von Ethylen und Monomeren, die eine Carboxygruppe aufweisen; Monomeren, die eine Anhydridgruppe aufweisen; Monomeren, die ein Säurechloridgruppe enthalten; Monomeren, die eine Estergruppe aufweisen; und Monomeren, die eine Isocyanatgruppe enthalten, ausgewählt sind.

**18.** Zusammensetzung nach einem der Ansprüche 15 bis 17, **dadurch gekennzeichnet, daß** das Polysiloxanmakromer ein Polysiloxan ist, das am Ende der Polysiloxankette oder in der Nähe des Kettenendes eine funktionelle Gruppe aufweist, die unter Alkoholen, Thiolen, Epoxy und primären und sekundären Aminen ausgewählt ist.

**19.** Zusammensetzung nach einem der Ansprüche 15 bis 18, **dadurch gekennzeichnet, daß** das Polysiloxanmakromer ein Polysiloxan ist, das der folgenden allgemeinen Formel (III) entspricht:

$$T\text{-}(CH_2)_s\text{-}Si\text{-}[\text{-}(OSiR^5R^6)_t\text{-}R^7]_y \qquad \text{(III)},$$

worin T unter $NH_2$, NHR', Epoxy, OH und SH ausgewählt ist; $R^5$, $R^6$, $R^7$ und R' unabhängig voneinander $C_{1-6}$-Alkyl, Phenyl, Benzyl, $C_{6-12}$-Alkylphenyl oder Wasserstoff bedeuten; s eine Zahl von 2 bis 100; t eine Zahl von 0 bis 1 000; und y eine Zahl von 1 bis 3 ist.

**20.** Zusammensetzung nach Anspruch 1 oder 2, **dadurch gekennzeichnet, daß** sie mindestens ein gepfropftes Siliconpolymer mit einem Polysiloxangerüst enthält, das mit nicht siliconhaltigen organischen Monomeren gepfropft ist, die eine Siliconhauptkette enthalten, auf die in der Kette sowie gegebenenfalls an mindestens einem ihrer Enden mindestens eine organische Gruppe gepfropft ist, die kein Silicon enthält.

**21.** Zusammensetzung nach Anspruch 20, **dadurch gekennzeichnet, daß** das gepfropfte Siliconpolymer mit einem Polysiloxangerüst, das mit nicht siliconhaltigen organischen Monomeren gepfropft ist, durch radikalische Copolymerisation mindestens eines nicht siliconhaltigen, anionischen, organischen Monomers, das eine ethylenische Doppelbindung aufweist, und/oder eines nicht siliconhaltigen, hydrophoben, organischen Monomers, das eine ethylenische Doppelbindung aufweist, und eines Polysiloxans, das in seiner Kette mindestens eine funktionelle Gruppe enthält, die mit den ethylenischen Doppelbindungen der nicht siliconhaltigen Monomere reagieren kann, hergestellt werden kann.

**22.** Zusammensetzung nach Anspruch 21, **dadurch gekennzeichnet, daß** das anionische, organische Monomer mit ethylenischer Doppelbindung einzeln oder in Form von Monomerengemischen unter den geradkettigen oder verzweigten, ungesättigten Carbonsäuren ausgewählt ist.

**23.** Zusammensetzung nach Anspruch 22, **dadurch gekennzeichnet, daß** das anionische, organische Monomer mit ethylenischer Doppelbindung einzeln oder in Form von Monomerengemischen unter Acrylsäure, Methacrylsäure, Maleinsäure, Maleinsäureanhydrid, Itaconsäure, Fumarsäure und Crotonsäure oder deren Alkalisalzen, Erdalkalisalzen oder Ammoniumsalzen oder deren Gemischen ausgewählt ist.

**24.** Zusammensetzung nach Anspruch 22, **dadurch gekennzeichnet, daß** das hydrophobe, organische Monomer mit ethylenischer Doppelbindung einzeln oder in Form von Monomerengemischen unter den Estern von Acrylsäure und Alkanolen und/oder Estern von Methacrylsäure und Alkanolen oder deren Gemischen ausgewählt ist, wobei die Alkanole vorzugsweise 1 bis 18 Kohlenstoffatome aufweisen.

**25.** Zusammensetzung nach Anspruch 24, **dadurch gekennzeichnet, daß** das hydrophobe, organische Monomer mit ethylenischer Doppelbindung einzeln oder in Form von Monomerengemischen unter Isooctyl(meth)acrylat, Isononyl(meth)acrylat, 2-Ethylhexyl(meth)acrylat, Lauryl(meth)acrylat, Isopentyl(meth)acrylat, n-Butyl(meth)acrylat, Isobutyl(meth)acrylat, Methyl(meth)acrylat, tert.-Butyl(meth)acrylat, Tridecyl(meth)acrylat und Stearyl(meth) acrylat ausgewählt ist.

**26.** Zusammensetzung nach einem der Ansprüche 20 bis 25, **dadurch gekennzeichnet, daß** das gepfropfte Siliconpolymer in der Siliconhauptkette mindestens eine organische Gruppe mit anionischen Eigenschaften enthält, die durch radikalische (Homo)polymerisation mindestens eines anionischen Monomers vom Typ einer ungesättigten, ganz oder teilweise in Form eines Salzes neutralisierten Carbonsäure erhalten wird.

**27.** Zusammensetzung nach einem der Ansprüche 20 bis 26, **dadurch gekennzeichnet, daß** das gepfropfte Siliconpolymer unter den Siliconpolymeren ausgewählt ist, die in ihrer Struktur die Einheit der folgenden Formel (IV) aufweisen:

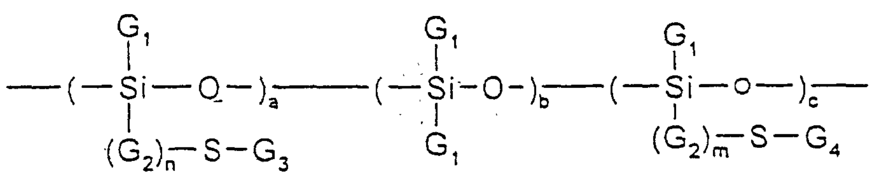 (IV),

worin die Gruppen $G_1$, die identisch oder voneinander verschieden sind, Wasserstoff, $C_{1-10}$-Alkyl oder Phenyl; die Gruppen $G_2$, die identisch oder voneinander verschieden sind, $C_{1-10}$-Alkylen; $G_3$ eine Polymergruppe, die aus der (Homo)polymerisation mindestens eines anionischen Monomers mit ethylenischer Doppelbindung resultiert; $G_4$ eine Polymergruppe, die aus der (Homo)polymerisation mindestens eines hydrophoben Monomers mit ethylenischer Doppelbindung resultiert; m und n 0 oder 1; a 0 oder eine ganze Zahl im Bereich von 1 bis 50; b eine ganze Zahl im Bereich von 10 bis 350; und c 0 oder eine ganze Zahl von 1 bis 50 bedeuten, mit der Maßgabe, daß einer der Parameter a oder c von 0 verschieden ist.

**28.** Zusammensetzung nach Anspruch 27, **dadurch gekennzeichnet, daß** die Einheit der Formel (IV) mindestens eine der folgenden Eigenschaften aufweist:

- die Gruppen $G_1$ bedeuten $C_{1-10}$-Alkyl;
- n ist nicht Null und die Gruppen $G_2$ bedeuten eine zweiwertige Gruppe mit 1 bis 3 Kohlenstoffatomen;
- $G_3$ bedeutet eine Polymergruppe, die aus der (Homo)polymerisation mindestens eines Monomers vom Typ einer Carbonsäure mit ethylenischer Doppelbindung resultiert;

- $G_4$ bedeutet eine Polymergruppe, die aus der (Homo)polymerisation mindestens eines Monomers vom Typ $C_{1-10}$-Alkyl(meth)-acrylat resultiert.

**29.** Zusammensetzung nach Anspruch 27 oder 28, **dadurch gekennzeichnet, daß** die Einheit der Formel (IV) gleichzeitig die folgenden Eigenschaften aufweist:

- die Gruppen $G_1$ bedeuten Methyl;
- n ist nicht Null und die Gruppen $G_2$ bedeuten Propylen;
- $G_3$ bedeutet eine Polymergruppe, die aus der (Homo)polymerisation von zumindest Acrylsäure und/oder Methacrylsäure resultiert;
- $G_4$ bedeutet eine Polymergruppe, die aus der (Homo)polymerisation von zumindest Isobutyl(meth)acrylat oder Methyl(meth)-acrylat resultiert.

**30.** Zusammensetzung nach einem der Ansprüche 20 bis 29, **dadurch gekennzeichnet, daß** das Zahlenmittel des Molekulargewichts des Polymers mit Polysiloxangerüst im Bereich von 10 000 bis 1 000 000 und noch bevorzugter etwa 10 000 bis 100 000 liegt.

**31.** Zusammensetzung nach einem der Ansprüche 1 bis 30, **dadurch gekennzeichnet, daß** das gepfropfte Siliconpolymer oder die gepfropften Siliconpolymere in Konzentrationen von 0,01 bis 20 Gew.-%, bezogen auf das Gesamtgewicht der Zusammensetzung, vorzugsweise im Bereich von 0,1 bis 15 Gew.-% und insbesondere im Bereich von 0,5 bis 10 Gew.-% verwendet werden.

**32.** Zusammensetzung nach einem der vorhergehenden Ansprüche, **dadurch gekennzeichnet, daß** das lineare Blockcopolymer der folgenden allgemeinen Formel entspricht:

$$([Y(R_2SiO)_aR'_2SiYO][(C_nH_{2n}O)_b])_c$$

worin bedeuten:

- die Gruppen R und R', die identisch oder voneinander verschieden sind, eine einwertige Kohlenwasserstoffgruppe, die keine aliphatische Doppelbindung aufweist,
- n eine ganze Zahl von 2 bis 4,
- a 5 oder eine ganze Zahl über 5,
- b 4 oder eine ganze Zahl über 4,
- c 4 oder eine ganze Zahl über 4,
- Y eine zweiwertige organische Gruppe, die über eine Kohlenstoff-Silicium-Bindung an das angrenzende Siliciumatom und über ein Sauerstoffatom an einen Polyoxyalkylenblock gebunden ist;

wobei:

- das mittlere Molekulargewicht jedes Siloxanblocks im Bereich von etwa 400 bis etwa 10 000 und jedes Polyoxyalkylenblocks im Bereich von etwa 300 bis etwa 10 000 liegt,
- die Siloxanblöcke etwa 10 bis etwa 95 Gew.-% des Blockcopolymers ausmachen, und
- das Zahlenmittel des Molekulargewichts des Blockcopolymers mindestens 3 000 beträgt.

**33.** Zusammensetzung nach Anspruch 32, **dadurch gekennzeichnet, daß** die Gruppen R und R' unter Methyl, Ethyl, Propyl, Butyl, Pentyl, Hexyl, Octyl, Decyl, Docecyl, Phenyl, Naphthyl, Benzyl, Phenylethyl, Tolyl, Xylyl und Cyclohexyl ausgewählt sind.

**34.** Zusammensetzung nach einem der Ansprüche 32 oder 33, **dadurch gekennzeichnet, daß** Y unter -R"-, -R"-CO-, -R"-NHCO-, -R"NH-CO-NH-R"'-NHCO oder -R"-OCONH-R"'-NHCO- ausgewählt ist, wobei R" Ethylen, Propylen oder Butylen und R"' $-C_6H_4-$, $-C_6H_4-C_6H_4-$, $-C_6H_4-CH_2-C_6H_4-$ und $-C_6H_4C-(CH_3)_2-C_6H_4-$ bedeutet.

**35.** Zusammensetzung nach einem der vorhergehenden Ansprüche, **dadurch gekennzeichnet, daß** die linearen Polysiloxan-Polyoxyalkylen-Blockcopolymere unter den Verbindungen der folgenden Formel ausgewählt sind:

$$[C_4H_8O(C_nH_{2n}O)_b\text{- }C_4H_8\text{ -}SiMe_2O(SiMe_2O)_aSiMe_2]_c$$

worin bedeuten: Me Methyl, n eine ganze Zahl von 2 bis 4, a und b 4 oder eine ganze Zahl über 4, und c 4 oder eine Zahl über 4.

**36.** Zusammensetzung nach Anspruch 35, **dadurch gekennzeichnet, daß** die linearen Polysiloxan-Polyoxyalkylen-Blockcopolymere als wiederkehrende Einheit aufweisen:

$$[\text{-}(SiMe_2O)_xSiMe_2\text{- }C_4H_8O\text{-}(C_2H_4O)_y(C_3H_6O)_z\text{-}C_4H_8\text{-}],$$

wobei x eine Zahl von 5 bis 15, y ein Zahl von 15 bis 30 und z eine Zahl von 20 bis 40 bedeutet.

**37.** Zusammensetzung nach einem der Ansprüche 1 oder 35, **dadurch gekennzeichnet, daß** das Blockcopolymer unter den folgenden Verbindungen ausgewählt ist:

$$[[(CH_3)_2SiO]_{41}(CH_3)_2SiCH_2CH(CH_3)CH_2\text{-}O(C_2H_4O)_{18}\text{-}(C_3H_6O)_{33}\text{-}CH_2CH(CH_3)CH_2]_{16.1}$$

$$[[(CH_3)_2SiO]_{31}(CH_3)_2SiCH_2CH(CH_3)CH_2\text{-}O(C_2H_4O)_{20}\text{-}(C_3H_6O)_{29}\text{-}CH_2CH(CH_3)CH_2]_{13.3}$$

$$[[(CH_3)_2SiO]_9(CH_3)_2SiCH_2CH(CH_3)CH_2\text{-}O(C_2H_4O)_{20}\text{-}(C_3H_6O)_{29}\text{-}CH_2CH(CH_3)CH_2]_{26.3}$$

$$[[(CH_3)_2SiO]_{16}(CH_3)_2SiCH_2CH(CH_3)CH_2\text{-}O(C_2H_4O)_{18}\text{-}(C_3H_6O)_{20}\text{-}CH_2CH(CH_3)CH_2]_{21.5}$$

$$[[(CH_3)_2SiO]_9(CH_3)_2SiCH_2CH(CH_3)CH_2\text{-}O(C_2H_4O)_5\text{-}CH_2CH(CH_3)CH_2]_{4.8}.$$

**38.** Zusammensetzung nach einem der vorhergehenden Ansprüche, **dadurch gekennzeichnet, daß** das lineare Blockcopolymer oder die linearen Blockcopolymere in einem Mengenanteil von 0,01 bis 20 Gew.-%, bezogen auf das Gesamtgewicht der Zusammensetzung, vorzugsweise 0,1 bis 15 Gew.-% und insbesondere 0,5 bis 10 Gew.-% vorliegen.

**39.** Zusammensetzung nach einem der vorhergehenden Ansprüche, **dadurch gekennzeichnet, daß** sie ferner mindestens einen Zusatzstoff enthält, der unter den Verdickungsmitteln, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen herkömmlich in der Kosmetik verwendeten Zusatzstoffen ausgewählt ist.

**40.** Zusammensetzung nach einem der vorhergehenden Ansprüche, **dadurch gekennzeichnet, daß** sie ein nicht siliconhaltiges, anionisches oder amphoteres Polymer in solubilisierter oder dispergierter Form enthält.

**41.** Zusammensetzung nach einem der vorhergehenden Ansprüche, **dadurch gekennzeichnet, daß** das kosmetisch oder dermatologisch akzeptable Medium aus Wasser oder einem Gemisch von Wasser und mindestens einem kosmetisch akzeptablen Lösungsmittel besteht.

**42.** Zusammensetzung nach Anspruch 41, **dadurch gekennzeichnet, daß** die kosmetisch akzeptablen Lösungsmittel unter Monoalkoholen, Polyalkoholen, Glykolethern, Fettsäureestern und deren Gemischen ausgewählt sind.

**43.** Zusammensetzung nach einem der vorhergehenden Ansprüche, **dadurch gekennzeichnet, daß** es sich bei den Keratinsubstanzen um Haar handelt.

**44.** Zusammensetzung nach einem der vorhergehenden Ansprüche, **dadurch gekennzeichnet, daß** sie als Gel, Milch, Creme, mehr oder weniger dickflüssige Lotion oder Schaum vorliegt.

**45.** Zusammensetzung nach einem der vorhergehenden Ansprüche, **dadurch gekennzeichnet, daß** es sich um ein Produkt zum Frisieren handelt.

**46.** Zusammensetzung nach einem der vorhergehenden Ansprüche, **dadurch gekennzeichnet, daß** es sich um ein Produkt für das Haar handelt, das unter den Haarwaschmitteln und Produkten für das Haar ausgewählt ist, die ausgespült oder nicht ausgespült werden oder vor oder nach einer Haarwäsche, Färbung, Entfärbung, Dauerwelle oder Entkräuselung aufgetragen werden.

**47.** Zusammensetzung nach einem der vorhergehenden Ansprüche, **dadurch gekennzeichnet, daß** sie in einem Zerstäuber, Pumpflakon oder Aerosolbehälter konfektioniert ist, um ein Spray, einen Lack oder einen Schaum zu erhalten.

**48.** Zusammensetzung nach einem der vorhergehenden Ansprüche, **dadurch gekennzeichnet, daß** das gepfropfte Siliconpolymer in dem kosmetisch oder dermatologisch akzeptablen Medium gelöst ist oder in Form einer wäßrigen Dispersion von Partikeln verwendet wird.

**49.** Nicht therapeutisches Verfahren zur Behandlung von Keratinsubstanzen und insbesondere menschlichem Haar, **dadurch gekennzeichnet, daß** es darin besteht, auf die Keratinsubstanzen eine Zusammensetzung nach einem der vorhergehenden Ansprüche aufzutragen und gegebenenfalls mit Wasser zu spülen.

## Claims

**1.** Cosmetic or dermatological composition intended for treating keratin substances, **characterized in that** it contains, in a cosmetically or dermatologically acceptable medium, at least one grafted silicone polymer comprising a polysiloxane portion and a portion consisting of a non-silicone organic chain, one of the two portions constituting the main chain of the polymer, the other being grafted onto the said main chain, and at least one copolymer having a linear polysiloxane/polyoxyalkylene block as repeating units.

**2.** Composition according to Claim 1, **characterized in that** the grafted silicone polymer is chosen from the group consisting of polymers having a non-silicone organic skeleton grafted with monomers containing a polysiloxane, polymers having a polysiloxane skeleton grafted with non-silicone organic monomers and mixtures thereof.

**3.** Composition according to Claim 1 or 2, **characterized in that** it contains at least one grafted silicone polymer containing a non-silicone organic skeleton, consisting of an organic main chain formed from organic monomers containing no silicone, on which is grafted, inside the said chain and optionally on at least one of its ends, at least one polysiloxane macromer.

**4.** Composition according to Claim 3, **characterized in that** the non-silicone organic monomers constituting the main chain of the grafted silicone polymer are chosen from the group consisting of monomers containing ethylenic unsaturation which are polymerizable via a radical route, monomers which are polymerizable by polycondensation and monomers which involve ring opening.

**5.** Composition according to any one of Claims 1 to 4, **characterized in that** it contains at least one silicone grafted copolymer comprising:

a) from 0 to 98% by weight of at least one lipophilic monomer (A) of low polarity containing ethylenic unsaturation of low polarity, which is polymerizable via a radical route;
b) from 0 to 98% by weight of at least one polar hydrophilic monomer (B) containing ethylenic unsaturation, which is copolymerizable with the (A)-type monomer(s);
c) from 0.01 to 50% by weight of at least one polysiloxane macromer (C) of general formula:

$$X(Y)_n Si(R)_{3-m} Z_m \qquad (I)$$

where:

X denotes a vinyl group which is copolymerizable with the monomers (A) and (B);
Y denotes a divalent bonding group;
R denotes a hydrogen, a $C_1$-$C_6$ alkyl or alkoxy or a $C_6$-$C_{12}$ aryl;
Z denotes a monovalent polysiloxane unit having a number-average molecular weight of at least 500;
n is 0 or 1 and m is an integer ranging from 1 to 3; the percentages being calculated relative to the total weight of the monomers (A), (B) and (C).

**6.** Composition according to Claim 5, **characterized in that** the lipophilic monomers (A) are chosen from the group consisting of acrylic or methacrylic acid esters of $C_1$-$C_{18}$ alcohols; styrene; polystyrene macromers; vinyl acetate; vinyl propionate; α-methylstyrene; tert-butylstyrene; butadiene; cyclohexadiene; ethylene; propylene; vinyltoluene; acrylic or methacrylic acid esters of 1,1-dihydroperfluoroalkanols or of homologues thereof; acrylic or methacrylic acid esters of ω-hydridofluoroalkanols; acrylic or methacrylic acid esters of fluoroalkylsulphoamido alcohols; acrylic or methacrylic acid esters of fluoroalkyl alcohols; acrylic or methacrylic acid esters of fluoroether alcohols; or mixtures thereof.

**7.** Composition according to Claim 6, **characterized in that** the lipophilic monomers (A) are chosen from the group consisting of n-butyl methacrylate, isobutyl methacrylate, tert-butyl acrylate, tert-butyl methacrylate, 2-ethylhexyl methacrylate, methyl methacrylate, 2-(N-butylperfluorooctanesulphonamido)ethyl acrylate, 2-(N-methylperfluorooctanesulphonamido)ethyl acrylate and mixtures thereof.

**8.** Composition according to any one of Claims 5 to 7, **characterized in that** the polar monomers (B) are chosen from the group consisting of acrylic acid, methacrylic acid, N,N-dimethylacrylamide, dimethylaminoethyl methacrylate, quaternized dimethylaminoethyl methacrylate, (meth) acrylamide, N-t-butylacrylamide, maleic acid, maleic anhydride and demiesters thereof, hydroxyalkyl (meth)acrylates, diallyldimethylammonium chloride, vinylpyrrolidone, vinyl ethers, maleimides, vinylpyridine, vinylimidazole, heterocyclic vinyl polar compounds, styrene sulphonate, allyl alcohol, vinyl alcohol and vinyl caprolactam, or mixtures thereof.

**9.** Composition according to Claim 8, **characterized in that** the polar monomers (B) are chosen from the group consisting of acrylic acid, N,N-dimethylacrylamide, dimethylaminoethyl methacrylate, quaternized dimethylaminoethyl methacrylate and vinylpyrrolidone, and mixtures thereof.

**10.** Composition according to any one of Claims 5 to 9, **characterized in that** the polysiloxane macromer (C) corresponds to the general formula (II) below:

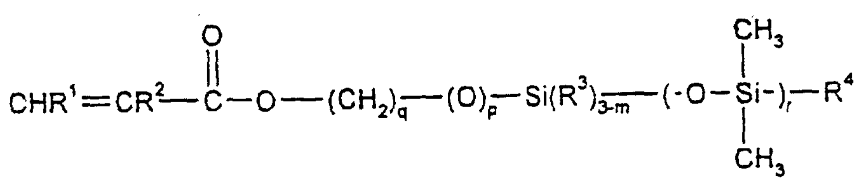 (II)

in which:

$R^1$ is hydrogen or -COOH;
$R^2$ is hydrogen, methyl or $-CH_2COOH$;
$R^3$ is $C_1$-$C_6$ alkyl, alkoxy or alkylamino, $C_6$-$C_{12}$ aryl or hydroxyl;
$R^4$ is $C_1$-$C_6$ alkyl, alkoxy or alkylamino, $C_6$-$C_{12}$ aryl or hydroxyl;
q is an integer from 2 to 6;
p is 0 or 1;
r is an integer from 5 to 700;
m is an integer from 1 to 3.

**11.** Composition according to any one of Claims 5 to 10, **characterized in that** the polysiloxane macromer (C) corresponds to the following general formula:

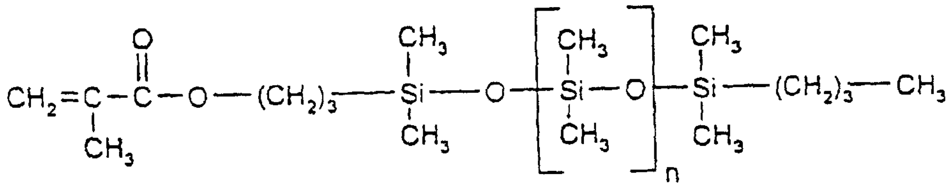

with n being an integer ranging from 5 to 700.

**12.** Composition according to any one of Claims 1 to 11, **characterized in that** it contains at least one copolymer which can be obtained by radical polymerization starting with a monomer mixture consisting of:

a) 60% by weight of tert-butyl acrylate;
b) 20% by weight of acrylic acid;
c) 20% by weight of silicone macromer of formula:

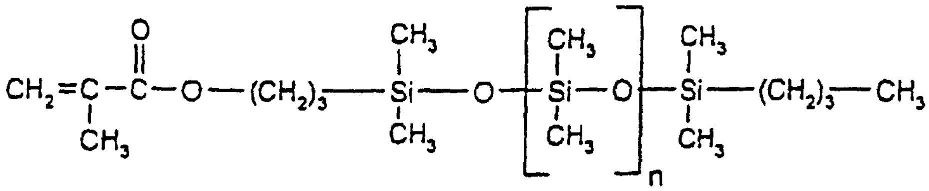

with n being an integer ranging from 5 to 700; the weight percentages being calculated relative to the total weight of the monomers.

**13.** Composition according to any one of Claims 1 to 11, **characterized in that** it contains at least one copolymer which can be obtained by radical polymerization starting with a monomer mixture consisting of:

a) 80% by weight of tert-butyl acrylate;
b) 20% by weight of silicone macromer of formula:

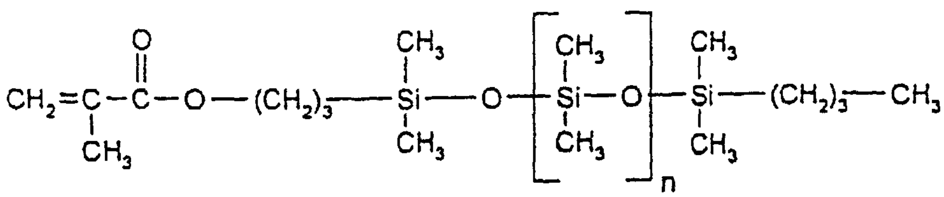

with n being an integer ranging from 5 to 700; the weight percentages being calculated relative to the total weight of the monomers.

**14.** Composition according to any one of Claims 5 to 13, **characterized in that** the polymer containing a non-silicone organic skeleton grafted with monomers containing a polysiloxane has a number-average molecular weight ranging from 10,000 to 2,000,000 and a glass transition temperature Tg or a crystalline melting point Tm of at least -20°C.

**15.** Composition according to any one of Claims 1 to 4, **characterized in that** it contains at least one polymer containing a non-silicone organic skeleton grafted with monomers containing a polysiloxane, which polymer can be obtained by reactive extrusion of a polysiloxane macromer having a terminal reactive function, with a polyolefin-type polymer containing reactive groups which can react with the terminal reactive function of the polysiloxane macromer in order to form a covalent bond allowing grafting of the silicone to the main chain of the polyolefin.

**16.** Composition according to Claim 15, **characterized in that** the reactive polyolefin is chosen from the group consisting of polyethylenes or polymers of ethylene-derived monomers containing reactive functions which can react

with the terminal function of the polysiloxane macromer.

**17.** Composition according to Claim 15 or 16, **characterized in that** the reactive polyolefin is chosen from the group consisting of copolymers of ethylene or of ethylene derivatives and of monomers chosen from those containing a carboxylic function; those containing an acid anhydride function; those containing an acid chloride function; those containing an ester function; those containing an isocyanate function.

**18.** Composition according to any one of Claims 15 to 17, **characterized in that** the polysiloxane macromer is a polysiloxane containing a functionalized group, at the end of the polysiloxane chain or close to the end of the said chain, chosen from the group consisting of alcohols, thiols, epoxy groups and primary and secondary amines.

**19.** Composition according to any one of Claims 15 to 18, **characterized in that** the polysiloxane macromer is a polysiloxane corresponding to the general formula (III):

$$T\text{-}(CH_2)_s\text{-}Si\text{-}[(O\text{-}SiR^5R^6)_t\text{-}R^7]_y \qquad (III)$$

in which T is chosen from the group consisting of $NH_2$, NHR', an epoxy, OH or SH function; $R^5$, $R^6$, $R^7$ and R', independently denote a $C_1$-$C_6$ alkyl, phenyl, benzyl, $C_6$-$C_{12}$ alkylphenyl or hydrogen; s is a number from 2 to 100; t is a number from 0 to 1000 and y is a number from 1 to 3.

**20.** Composition according to Claim 1 or 2, **characterized in that** it contains at least one grafted silicone polymer containing a polysiloxane skeleton grafted with non-silicone organic monomers, comprising a polysiloxane main chain on which is grafted, inside the said chain as well as, optionally, on at least one of its ends, at least one organic group containing no silicone.

**21.** Composition according to Claim 20, **characterized in that** the polymer containing a polysiloxane skeleton grafted with non-silicone organic monomers can be obtained by radical copolymerization between, on the one hand, at least one non-silicone anionic organic monomer having ethylenic unsaturation and/or a non-silicone hydrophobic organic monomer having ethylenic unsaturation, and, on the other hand, a polysiloxane having in its chain at least one functional group capable of reacting with the said ethylenic unsaturations of the said non-silicone monomers.

**22.** Composition according to Claim 21, **characterized in that** the anionic organic monomer containing ethylenic unsaturation is chosen, alone or in the form of a monomer mixture, from linear or branched, unsaturated carboxylic acids.

**23.** Composition according to Claim 22, **characterized in that** the anionic organic monomer containing ethylenic unsaturation is chosen, alone or in the form of a monomer mixture, from acrylic acid, methacrylic acid, maleic acid, maleic anhydride, itaconic acid, fumaric acid and crotonic acid or alkali-metal, alkaline-earth metal or ammonium salts thereof or mixtures thereof.

**24.** Composition according to Claim 22, **characterized in that** the hydrophobic organic monomer containing ethylenic unsaturation is chosen, alone or as a monomer mixture, from acrylic acid esters of alkanol and/or methacrylic acid esters of alkanol, the alkanol preferably being $C_1$-$C_{18}$.

**25.** Composition according to Claim 24, **characterized in that** the hydrophobic organic monomer containing ethylenic unsaturation is chosen, alone or as a monomer mixture, from the group consisting of isooctyl (meth)acrylate, isononyl (meth)acrylate, 2-ethylhexyl (meth)acrylate, lauryl (meth)acrylate, isopentyl (meth)acrylate, n-butyl (meth) acrylate, isobutyl (meth)acrylate, methyl (meth)acrylate, tert-butyl (meth)acrylate, tridecyl (meth)acrylate and stearyl (meth)acrylate.

**26.** Composition according to any one of Claims 20 to 25, **characterized in that** the grafted silicone polymer comprises, on the main silicone chain, at least one organic group of anionic nature obtained by the radical (homo)polymerization of at least one anionic monomer of unsaturated carboxylic acid type, which is partially or totally neutralized in the form of a salt.

**27.** Composition according to any one of Claims 20 to 26, **characterized in that** the grafted silicone polymer is chosen

from silicone polymers containing in their structure the unit of formula (IV) below:

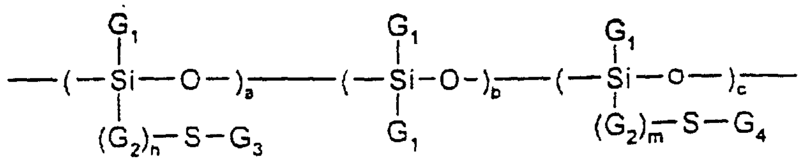

(IV)

in which the radicals $G_1$, which may be identical or different, represent hydrogen or a $C_1$-$C_{10}$ alkyl radical or alternatively a phenyl radical; the radicals $G_2$, which may be identical or different, represent a $C_1$-$C_{10}$ alkylene group; $G_3$ represents a polymer residue resulting from the (homo)polymerization of at least one anionic monomer containing ethylenic unsaturation; $G_4$ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350, c is an integer ranging from 0 to 50; with the proviso that one of the parameters a and c is other than 0.

**28.** Composition according to Claim 27, **characterized in that** the unit of formula (IV) has at least one of the following characteristics:

- the radicals $G_1$ denote a $C_1$-$C_{10}$ alkyl radical;
- n is non-zero and the radicals $G_2$ represent a divalent $C_1$-$C_3$ radical;
- $G_3$ represents a polymeric radical resulting from the (homo)polymerization of at least one monomer of the carboxylic acid type containing ethylenic unsaturation;
- $G_4$ represents a polymeric radical resulting from the (homo)polymerization of at least one monomer of the $C_1$-$C_{10}$ alkyl (meth)acrylate type.

**29.** Composition according to Claim 27 or 28, **characterized in that** the unit of formula (IV) simultaneously has the following characteristics:

- the radicals $G_1$ denote a methyl radical;
- n is non-zero and the radicals $G_2$ represent a propylene radical;
- $G_3$ represents a polymeric radical resulting from the (homo)polymerization of at least one acrylic acid and/or methacrylic acid;
- $G_4$ represents a polymeric radical resulting from the (homo)polymerization of at least isobutyl or methyl (meth) acrylate.

**30.** Composition according to any one of Claims 20 to 29, **characterized in that** the number-average molecular mass of the polymer containing a polysiloxane skeleton grafted with non-silicone organic monomers ranges approximately from 10,000 to 1,000,000 and even more preferably approximately from 10,000 to 100,000.

**31.** Composition according to any one of Claims 1 to 30, **characterized in that** the grafted silicone polymer(s) is (are) used in an amount ranging from 0.01 to 20% by weight relative to the total weight of the composition, preferably from 0.1 to 15% by weight and more particularly from 0.5 to 10% by weight.

**32.** Composition according to any one of the preceding claims, **characterized in that** the linear block copolymer corresponds to the general formula:

$$([Y(R_2SiO)_aR'_2SiYO]\ [(C_nH_{2n}O)_b])c$$

in which:

- R and R', which may be identical or different, represent a monovalent hydrocarbon radical containing no aliphatic unsaturation,
- n is an integer ranging from 2 to 4,
- a is an integer greater than or equal to 5,

- b is an integer greater than or equal to 4,
- c is an integer greater than or equal to 4,
- Y represents a divalent organic group which is linked to the adjacent silicon atom via a carbon-silicon bond and to a polyoxyalkylene block via an oxygen atom,
- the average molecular weight of each siloxane block is between about 400 and about 10,000, that of each polyoxyalkylene block being between about 300 and about 10,000,
- the siloxane blocks represent from about 10% to about 95% of the weight of the block copolymer,
- the average molecular weight of the block copolymer being at least 3000.

**33.** Composition according to Claim 32, **characterized in that** R and R' are chosen from the group consisting of methyl, ethyl, propyl, butyl, pentyl, hexyl, octyl, decyl, dodecyl, phenyl, naphthyl, benzyl, phenylethyl, tolyl, xylyl and cyclohexyl radicals.

**34.** Composition according to either of Claims 32 and 33, **characterized in that** Y is chosen from the group consisting of -R"-, -R"-CO-, -R"-NHCO-, R"-NH-CONH-R" 'NHCO and R"-OCONH-R'"-NHCO-, where R" represents an ethylene, propylene or butylene radical and R'" represents a $-C_6H_4-$, $-C_6H_4-C_6H_4-$, $-C_6H_4-CH_2-C_6H_4$ - or $-C_6H_4-CH(CH_3)_2-C_6H_4-$ group.

**35.** Composition according to any one of the preceding claims, **characterized in that** the linear polysiloxane/polyoxyalkylene block copolymers are chosen from those of formula:

$$[C_4H_8(C_nH_{2n}O)_b-C_4H_8-SiMe_2O(SiMe_2O)_aSiMe_2]_c$$

where Me denotes methyl, n is an integer from 2 to 4, a and b are integers greater than or equal to 4 and c is a number greater than or equal to 4.

**36.** Composition according to Claim 35, **characterized in that** the linear polysiloxane/polyoxyalkylene block copolymers have the repeating unit whose structure is:

$$[-(SiMe_2O)_xSiMe_2-C_4H_8O-(C_2H_4O)_y-(C_3H_6O)_z-C_4H_8-]$$

where x is a number between 5 and 15 inclusive, y is a number between 15 and 30 inclusive and z is a number between 20 and 40.

**37.** Composition according to any one of Claims 1 to 35, **characterized in that** the block copolymer is chosen from the group comprising the following compounds:

$$[[(CH_3)_2SiO]_{41}(CH_3)_2SiCH_2CH(CH_3)CH_2-O(C_2H_4O)_{18}-(C_3H_6O)_{33}CH_2CH(CH_3)CH_2]_{16.1}$$

$$[[(CH_3)_2SiO]_{31}(CH_3)_2SiCH_2CH(CH_3)CH_2-O(C_2H_4O)_{20}-(C_3H_6O)_{29}CH_2CH(CH_3)CH_2]_{13.3}$$

$$[[(CH_3)_2SiO]_{9}(CH_3)_2SiCH_2CH(CH_3)CH_2-O(C_2H_4O)_{20}-(C_3H_6O)_{29}CH_2CH(CH_3)CH_2]_{26.3}$$

$$[[(CH_3)_2SiO]_{16}(CH_3)_2SiCH_2CH(CH_3)CH_2-O(C_2H_4O)_{18}-(C_3H_6O)_{20}CH_2CH(CH_3)CH_2]_{21.5}$$

$$[[(CH_3)_2SiO]_{9}(CH_3)_2SiCH_2CH(CH_3)CH_2-O(C_2H_4O)_{5}-CH_2CH(CH_3)CH_2]_{4.8}$$

**38.** Composition according to any one of the preceding claims, **characterized in that** the linear block copolymer(s) is (are) present in concentrations ranging from 0.01 to 20% by weight relative to the total weight of the composition, preferably from 0.1 to 15% by weight and more particularly from 0.5 to 10% by weight.

**39.** Composition according to any one of the preceding claims, **characterized in that** it also contains at least one additive chosen from the group consisting of thickeners, fatty acid esters, fatty acid esters of glycerol, silicones, surfactants, fragrances, preserving agents, sunscreens, proteins, vitamins, polymers, plant, animal, mineral or synthetic oils or any other additive conventionally used in the cosmetic field.

**40.** Composition according to any one of the preceding claims, **characterized in that** it contains a non-silicone anionic or amphoteric polymer in solubilized form or in dispersed form.

**41.** Composition according to any one of the preceding claims, **characterized in that** the cosmetically or dermatologically acceptable medium consists of water or a mixture of water and at least one cosmetically acceptable solvent.

**42.** Composition according to Claim 41, **characterized in that** the cosmetically acceptable solvents are chosen from the group consisting of monoalcohols, polyalcohols, glycol ethers and fatty acid esters, and mixtures thereof.

**43.** Composition according to any one of the preceding claims, **characterized in that** the keratin substance is hair.

**44.** Composition according to any one of the preceding claims, **characterized in that** it is in the form of a gel, a milk, a cream, a relatively thickened lotion or a mousse.

**45.** Composition according to any one of the preceding claims, **characterized in that** it is a hairstyling product.

**46.** Composition according to any one of the preceding claims, **characterized in that** it is a hair product chosen from the group consisting of shampoos, rinse-out or leave-in hair products, to be applied before or after shampooing, dyeing, bleaching, permanent-waving or straightening the hair.

**47.** Composition according to any one of the preceding claims; **characterized in that** it is packaged in the form of a vaporizer or a pump-dispenser bottle or alternatively in an aerosol container in order to obtain a spray, a lacquer or a mousse.

**48.** Composition according to any one of the preceding claims, **characterized in that** the grafted silicone polymer is dissolved in the cosmetically or dermatologically acceptable medium or used in the form of an aqueous dispersion of particles.

**49.** Non-therapeutic process for treating keratin substances, in particular human hair, **characterized in that** it consists in applying a composition as defined according to any one of the preceding claims to the said substances and then optionally in rinsing with water.